# EUROPEAN PATENT SPECIFICATION

(11) **EP 2 667 895 B1**
(45) Date of publication and mention of the grant of the patent: **13.03.2019**
(21) Application number: 12714046.5
(22) Date of filing: 19.01.2012
(51) Int. Cl.: A61K 39/395, A61P 29/00, C07K 16/28

(54) **USE OF AT LEAST ONE P75NTR RECEPTOR INHIBITOR, ALONE OR IN ASSOCIATION WITH AT LEAST ONE TRKA RECEPTOR ACTIVATOR, OR OF AT LEAST ONE TRKA RECEPTOR ACTIVATOR, FOR THE TREATMENT OF CHRONIC INFLAMMATORY DISEASES**
VERWENDUNG VON MINDESTENS EINEM P75NTR-REZEPTORHEMMER, ALLEIN ODER IN ZUSAMMENHANG MIT MINDESTENS EINEM TRKA-REZEPTORAKTIVATOR, ODER VON MINDESTENS EINEM TRKA-REZEPTORAKTIVATOR ZUR BEHANDLUNG CHRONISCHER ENTZÜNDUNGSERKRANKUNGEN
UTILISATION D'AU MOINS UN INHIBITEUR DU RÉCEPTEUR P75NTR, SEUL OU EN ASSOCIATION AVEC AU MOINS UN ACTIVATEUR DU RÉCEPTEUR TRKA, OU D'AU MOINS UN ACTIVATEUR DU RÉCEPTEUR TRKA, POUR LE TRAITEMENT DE MALADIES INFLAMMATOIRES CHRONIQUES

(30) Priority: 24.01.2011 IT RM20110024
(43) Date of publication of application: 04.12.2013
(73) Proprietor: Ospedale Pediatrico Bambino Gesù, 00165 Roma (IT); Consiglio Nazionale delle Ricerche, 00185 Roma (IT)
(72) Inventor: BRACCI LAUDIERO, Luisa, 00193 Roma (IT); DE BENEDETTI, Fabrizio, 00165 Roma (IT)
(74) Representative: Gitto, Serena
(86) International application number: PCT/IT2012/000018
(87) International publication number: WO 2012/101664

(56) References cited:
- WO-A1-02/096458
- MICERA ALESSANDRA ET AL: "Nerve growth factor has a modulatory role on human primary fibroblast cultures derived from vernal keratoconjunctivitis-affected conjunctiva", MOLECULAR VISION, MOLECULAR VISION, SN, ATLANTA, vol. 13, 21 June 2007 (2007-06-21), pages 981-987, XP009099723, ISSN: 1090-0535
- BARTHEL CHRISTIAN ET AL: "Nerve growth factor and receptor expression in rheumatoid arthritis and spondyloarthritis", ARTHRITIS RESEARCH AND THERAPY, BIOMED CENTRAL, LONDON, GB, vol. 11, no. 3, 2 June 2009 (2009-06-02), page R82, XP021061237, ISSN: 1478-6354, DOI: DOI:10.1186/AR2716
- UGOLINI GABRIELE ET AL: "The function neutralizing anti-TrkA antibody MNAC13 reduces inflammatory and neuropathic pain", PROCEEDINGS OF THE NATIONAL ACADEMY OF SCIENCES, NATIONAL ACADEMY OF SCIENCES, WASHINGTON, DC; US, vol. 104, no. 8, 20 February 2007 (2007-02-20), pages 2985-2990, XP002523974, ISSN: 0027-8424, DOI: DOI:10.1073/PNAS.0611253104
- PETERSEN ET AL: "Nerve growth factor regulates the expression of bradykinin binding sites on adult sensory neurons via the neurotrophin receptor p75", NEUROSCIENCE, NEW YORK, NY, US, vol. 83, no. 1, 1 January 1998 (1998-01-01), pages 161-168, XP022255890, ISSN: 0306-4522, DOI: DOI:10.1016/S0306-4522(97)00374-6
- FREUND-MICHEL ET AL: "The nerve growth factor and its receptors in airway inflammatory diseases", PHARMACOLOGY AND THERAPEUTICS, ELSEVIER, GB, vol. 117, no. 1, 5 December 2007 (2007-12-05), pages 52-76, XP022375432, ISSN: 0163-7258, DOI: DOI:10.1016/J.PHARMTHERA.2007.07.003
- BULL H A ET AL: "Expression of nerve growth factor receptors in cutaneous inflammation", BRITISH JOURNAL OF DERMATOLOGY, vol. 139, no. 5, November 1998 (1998-11), pages 776-783, XP002643307, ISSN: 0007-0963
- SMRITI K. RAYCHAUDHURI ET AL: "NGF and Its Receptor System", ANNALS OF THE NEW YORK ACADEMY OF SCIENCES, vol. 1173, no. 1, 1 September 2009 (2009-09-01), pages 470-477, XP55239973, US ISSN: 0077-8923, DOI: 10.1111/j.1749-6632.2009.04652.x
- PELESHOK J ET AL: "FUNCTIONAL MIMETICS OF NEUROTROPHINS AND THEIR RECEPTORS", BIOCHEMICAL SOCIETY TRANSACTIONS, PORTLAND PRESS LTD, GB, vol. 34, no. PT 4, 1 August 2006 (2006-08-01), pages 612-617, XP009084092, ISSN: 0300-5127, DOI: 10.1042/BST0340612
- LEBRUN-JULIEN F ET AL: "Inhibition of p75<NTR> in glia potentiates TrkA-mediated survival of injured retinal ganglion cells", MOLECULAR AND CELLULAR NEUROSCIENCES, SAN DIEGO, US, vol. 40, no. 4, 1 April 2009 (2009-04-01), pages 410-420, XP026072277, ISSN: 1044-7431, DOI: 10.1016/J.MCN.2008.12.005 [retrieved on 2009-03-25]
- M. YAAR ET AL: "A cyclic peptide that binds p75 NTR protects neurones from beta amyloid (1?40)-induced cell death", NEUROPATHOLOGY AND APPLIED NEUROBIOLOGY., vol. 33, no. 5, 1 October 2007 (2007-10-01), pages 533-543, XP055340244, GB ISSN: 0305-1846, DOI: 10.1111/j.1365-2990.2007.00844.x
- MINA YAAR ET AL: "p75NTR Antagonistic Cyclic Peptide Decreases the Size of Î Amyloid-Induced Brain Inflammation", CELLULAR AND MOLECULAR NEUROBIOLOGY, KLUWER ACADEMIC PUBLISHERS-PLENUM PUBLISHERS, NE, vol. 28, no. 8, 19 September 2008 (2008-09-19), pages 1027-1031, XP019640502, ISSN: 1573-6830, DOI: 10.1007/S10571-008-9298-6
- MALIARTCHOUK S ET AL: "A DESIGNED PEPTIDOMIMETIC AGONISTIC LIGAND OF TRKA NERVE GROWTH FACTOR RECEPTORS", MOLECULAR PHARMACOLOGY, AMERICAN SOCIETY FOR PHARMACOLOGY AND EXPERIMENTAL THERAPEUTICS, US, vol. 57, no. 2, 1 February 2000 (2000-02-01), pages 385-391, XP001000324, ISSN: 0026-895X
- S. MAHAPATRA ET AL: "Identification of Critical Residues within the Conserved and Specificity Patches of Nerve Growth Factor Leading to Survival or Differentiation", JOURNAL OF BIOLOGICAL CHEMISTRY, vol. 284, no. 48, 27 November 2009 (2009-11-27), pages 33600-33613, XP055340260, US ISSN: 0021-9258, DOI: 10.1074/jbc.M109.058420
- Y. BAI ET AL: "Chronic and Acute Models of Retinal Neurodegeneration TrkA Activity Are Neuroprotective whereas p75NTR Activity Is Neurotoxic through a Paracrine Mechanism", JOURNAL OF BIOLOGICAL CHEMISTRY, vol. 285, no. 50, 13 October 2010 (2010-10-13), pages 39392-39400, XP055340264, US ISSN: 0021-9258, DOI: 10.1074/jbc.M110.147801
- Katharyn Spiegel ET AL: "PD-90780, a Nonpeptide Inhibitor of Nerve Growth Factor's Binding to the P75 NGF Receptor", , 14 December 1995 (1995-12-14), pages 488-494, XP055340267, DOI: http://dx.doi.org/10.1006/bbrc.1995.2802 Retrieved from the Internet: URL:http://www.sciencedirect.com/science/a rticle/pii/S0006291X85728021/pdf?md5=35922 1b304bf6c10ca032da8c0cd853c&pid=1-s2.0-S00 06291X85728021-main.pdf [retrieved on 2017-01-30]
- A. COLQUHOUN: "Differential Activity of the Nerve Growth Factor (NGF) Antagonist PD90780 [7-(Benzolylamino)-4,9-dihydro-4-methyl-9- oxo-pyrazolo[5,1-b]quinazoline-2-carboxyli c Acid] Suggests Altered NGF-p75NTR Interactions in the Presence of TrkA", JOURNAL OF PHARMACOLOGY AND EXPERIMENTAL THERAPEUTICS, vol. 310, no. 2, 9 April 2004 (2004-04-09) , pages 505-511, XP055340272, US ISSN: 0022-3565, DOI: 10.1124/jpet.104.066225
- S.-W. JANG ET AL: "Gambogic amide, a selective agonist for TrkA receptor that possesses robust neurotrophic activity, prevents neuronal cell death", PROCEEDINGS OF THE NATIONAL ACADEMY OF SCIENCES, vol. 104, no. 41, 9 October 2007 (2007-10-09), pages 16329-16334, XP055340274, US ISSN: 0027-8424, DOI: 10.1073/pnas.0706662104
- MARIANA PEHAR ET AL: "Modulation of p75 NTR -dependent motor neuron death by a small non-peptidyl mimetic of the neurotrophin loop 1 domain", EUROPEAN JOURNAL OF NEUROSCIENCE., vol. 24, no. 6, 1 September 2006 (2006-09-01), pages 1575-1580, XP055340283, GB ISSN: 0953-816X, DOI: 10.1111/j.1460-9568.2006.05040.x
- S. M. MASSA: "Small, Nonpeptide p75NTR Ligands Induce Survival Signaling and Inhibit proNGF-Induced Death", JOURNAL OF NEUROSCIENCE, vol. 26, no. 20, 17 May 2006 (2006-05-17), pages 5288-5300, XP055057505, ISSN: 0270-6474, DOI: 10.1523/JNEUROSCI.3547-05.2006
- FRANK LONGO ET AL: "Small Molecule Modulation of p75 Neurotrophin Receptor Functions", CNS & NEUROLOGICAL DISORDERS, vol. 7, no. 1, 1 February 2008 (2008-02-01), pages 63-70, XP055340289, NL ISSN: 1871-5273, DOI: 10.2174/187152708783885093
- LESAUTEUR L ET AL: "POTENT HUMAN P140-TRKA AGONISTS DERIVED FROM AN ANTI-RECEPTOR MONOCLONAL ANTIBODY", JOURNAL OF NEUROSCIENCE, SOCIETY FOR NEUROSCIENCE, US, vol. 16, no. 4, 15 February 1996 (1996-02-15), pages 1308-1316, XP000645295, ISSN: 0270-6474
- ROGERS M L ET AL: "Functional monoclonal antibodies to p75 neurotrophin receptor raised in knockout mice", JOURNAL OF NEUROSCIENCE METHODS, ELSEVIER SCIENCE PUBLISHER B.V., AMSTERDAM, NL, vol. 158, no. 1, 15 November 2006 (2006-11-15), pages 109-120, XP024997155, ISSN: 0165-0270, DOI: 10.1016/J.JNEUMETH.2006.05.022 [retrieved on 2006-11-15]
- A. H. ROSS ET AL: "Characterization of nerve growth factor receptor in neural crest tumors using monoclonal antibodies.", PROCEEDINGS OF THE NATIONAL ACADEMY OF SCIENCES, vol. 81, no. 21, 1 November 1984 (1984-11-01), pages 6681-6685, XP055340292, US ISSN: 0027-8424, DOI: 10.1073/pnas.81.21.6681
- WESKAMP G ET AL: "Evidence that biological activity of NGF is mediated through a novel subclass of high affinity receptors", NEURON, CELL PRESS, US, vol. 6, no. 4, 1 April 1991 (1991-04-01), pages 649-663, XP027463704, ISSN: 0896-6273, DOI: 10.1016/0896-6273(91)90067-A [retrieved on 1991-04-01]
- TUVERI M ET AL: "NGF, a useful tool in the treatment of chronic vasculitic ulcers in rheumatoid arthritis", THE LANCET, THE LANCET PUBLISHING GROUP, GB, vol. 356, no. 9243, 18 November 2000 (2000-11-18), pages 1739-1740, XP004813506, ISSN: 0140-6736, DOI: 10.1016/S0140-6736(00)03212-8
- ALOE L ET AL: "The topical application of nerve growth factor as a pharmacological tool for human corneal and skin ulcers", PHARMACOLOGICAL RESEARCH, ACADEMIC PRESS, LONDON, GB, vol. 57, no. 4, 1 April 2008 (2008-04-01), pages 253-258, XP022637158, ISSN: 1043-6618, DOI: 10.1016/J.PHRS.2008.01.010 [retrieved on 2008-02-02]
- RITA LEVI-MONTALCINI ET AL: "DESTRUCTION OF THE SYMPATHETIC GANGLIA IN MAMMALS BY AN ANTISERUM TO A NERVE-GROWTH PROTEIN", PNAS, vol. 46, 4 January 1960 (1960-01-04), pages 384-391, XP055351278,
- I ALOE ET AL: "Somatic and behavioural postnatal effects of fetal injections of nerve growth factor antibodies in the rat", NATURE, 4 June 1981 (1981-06-04), pages 413-415, XP055352042,
- Rm Lindsay ET AL: "Nerve growth factor regulates expression of neuropeptide genes in adult sensory neurons", Nature, 26 January 1989 (1989-01-26), pages 362-364, XP055352048, Retrieved from the Internet: URL:http://www.nature.com/nature/journal/v 337/n6205/pdf/337362a0.pdf [retrieved on 2017-03-07]
- V M K VERGE ET AL: "Differential Influence of Nerve Growth Factor on Neuropeptide Expression in vim: A Novel Role in Peptide Suppression in Adult Sensory Neurons", THE JOURNAL OF NEUROSCIENCE GRAFSTEIN, vol. 15, no. 6, 1 March 1995 (1995-03-01), pages 2081-2096, XP55352054,
- HOYLE GARY W ET AL: "Hyperinnervation of the airways in transgenic mice overexpressing nerve growth factor", AMERICAN JOURNAL OF RESPIRATORY CELL AND MOLECULAR BIOLOGY, AMERICAN LUNG ASSOCIATION, NEW YORK, NY, US, vol. 18, no. 2, 1 February 1998 (1998-02-01), pages 149-157, XP002592928, ISSN: 1044-1549
- GEORG PONGRATZ ET AL: "Role of peripheral nerve fibres in acute and chronic inflammation in arthritis", NATURE REVIEWS RHEUMATOLOGY, vol. 9, no. 2, 13 November 2012 (2012-11-13), pages 117-126, XP055352075, GB ISSN: 1759-4790, DOI: 10.1038/nrrheum.2012.181
- LUIGI ALOE ET AL: "Nerve growth factor in the synovial fluid of patients with chronic arthritis", ARTHRITIS & RHEUMATISM, vol. 35, no. 3, 1 March 1992 (1992-03-01), pages 351-355, XP055305001, US ISSN: 0004-3591, DOI: 10.1002/art.1780350315
- LAUDIERO L B ET AL: "Multiple sclerosis patients express increased levels of @b-nerve growth factor in cerebrospinal fluid", NEUROSCIENCE LETTERS, ELSEVIER, AMSTERDAM, NL, vol. 147, no. 1, 23 November 1992 (1992-11-23), pages 9-12, XP024362718, ISSN: 0304-3940, DOI: 10.1016/0304-3940(92)90762-V [retrieved on 1992-11-23]
- F FALCINI ET AL: "Increased circulating nerve growth factor is directly correlated with disease activity in juvenile chronic arthritis.", ANNALS OF THE RHEUMATIC DISEASES, vol. 55, no. 10, 1 October 1996 (1996-10-01), pages 745-748, XP055352078, GB ISSN: 0003-4967, DOI: 10.1136/ard.55.10.745
- MANNI L AND ALOE L: "Role of IL-1 beta and TNF-alpha in the regulation of NGF in experimentally induced arthritis in mice. - PubMed - NCBI", RHEUMATOL. INT., 1 January 1998 (1998-01-01), XP055352079,
- Wu Z ET AL: "Immunohistochemical study of NGF and its receptors in the synovial membrane of the ankle joint of adjuvant-induced arthritic rats. - PubMed - NCBI", , 1 December 2000 (2000-12-01), pages 453-459, XP055352082, Retrieved from the Internet: URL:https://www.ncbi.nlm.nih.gov/pubmed/11 201606 [retrieved on 2017-03-07]
- BIERL M A ET AL: "'Mature' nerve growth factor is a minor species in most peripheral tissues", NEUROSCIENCE LETTERS, ELSEVIER, AMSTERDAM, NL, vol. 380, no. 1-2, 20 May 2005 (2005-05-20), pages 133-137, XP027653619, ISSN: 0304-3940 [retrieved on 2005-05-20]
- YI XIA ET AL: "Presence of proNGF-Sortilin Signaling Complex in Nigral Dopamine Neurons and Its Variation in Relation to Aging, Lactacystin and 6-OHDA Insults", INTERNATIONAL JOURNAL OF MOLECULAR SCIENCES, vol. 14, no. 7, 8 July 2013 (2013-07-08), pages 14085-14104, XP055352084, DOI: 10.3390/ijms140714085
- ALOE L ET AL: "THE SYNOVIUM OF TRANSGENIC ARTHRITIC MICE EXPRESSING HUMAN TUMOR NECROSIS FACTORCONTAINS A HIGH LEVEL OF NERVE GROWTH FACTOR", GROWTH FACTORS, HARWOOD ACADEMIC PUBLISHERS GMBH, XX, vol. 9, 1 January 1993 (1993-01-01), pages 149-155, XP008070582, ISSN: 0897-7194
- SOLIGO MARZIA ET AL: "The mature/pro nerve growth factor ratio is decreased in the brain of diabetic rats: Analysis by ELISA methods", BRAIN RESEARCH, vol. 1624, 2015, pages 455-468, XP029290543, ISSN: 0006-8993, DOI: 10.1016/J.BRAINRES.2015.08.005
- M. FLORENCIA IULITA ET AL: "Nerve growth factor metabolic dysfunction in Alzheimer's disease and Down syndrome", TRENDS IN PHARMACOLOGICAL SCIENCES., vol. 35, no. 7, 1 July 2014 (2014-07-01), pages 338-348, XP055352086, GB ISSN: 0165-6147, DOI: 10.1016/j.tips.2014.04.010
- M. VOLOSIN ET AL: "Interaction of Survival and Death Signaling in Basal Forebrain Neurons: Roles of Neurotrophins and Proneurotrophins", JOURNAL OF NEUROSCIENCE, vol. 26, no. 29, 19 July 2006 (2006-07-19) , pages 7756-7766, XP55352087, US ISSN: 0270-6474, DOI: 10.1523/JNEUROSCI.1560-06.2006
- YAN-JIANG WANG ET AL: "Effects of proNGF on Neuronal Viability, Neurite Growth and Amyloid-beta Metabolism", NEUROTOXICITY RESEARCH, vol. 17, no. 3, 13 August 2009 (2009-08-13), pages 257-267, XP55352091, CH ISSN: 1029-8428, DOI: 10.1007/s12640-009-9098-x

## Description

The present invention concerns the use of at least one p75NTR receptor inhibitor, alone or in association with at least one TrkA receptor activator, or at least one TrkA receptor activator, for the treatment of chronic inflammatory diseases. In particular, the invention concerns the use of inhibitors (antagonists) of p75NTR receptor and/or of activators (agonists) of TrkA receptor, as for example NGF, for the treatment of chronic inflammatory diseases of autoimmune or auto-inflammatory origin as, for example, rheumatoid arthritis, juvenile idiopathic arthritis, Crohn's disease, psoriasis, multiple sclerosis, systemic lupus erythematosus.

Chronic inflammatory diseases are a group of diseases characterized by chronic inflammation leading to tissue damage and loss of target tissue function. The causes of these diseases are not known in detail. Current therapies are based on the use of not steroidal anti-inflammatory drugs, cortisone based and immunosuppressive drugs. Treatments are effective only for a low percentage of patients and are side-effect burdened. More recently the introduction of some biological drugs has improved the response percentages. It is however apparent that a remarkable percentage of patients suffering from chronic inflammatory diseases does not display a satisfactory clinical response and also these drugs are burdened by side-effects both in short and long term.

In the light of above it is therefore apparent the need to provide for new therapies for treatment and prevention of chronic inflammatory diseases suitable to overcome the disadvantages of known art.

During the last years a lot of data demonstrating the role of Toll-like (TLRs) receptors in the onset of inflammatory and autoimmune diseases are reported. The dysregulation of Toll-like receptor associated signal pathways results in an increase of the synthesis of inflammatory cytokines and chemokines that, in association with alterations in the endogenous anti-inflammatory mechanisms, results in a chronic inflammatory process (1). Among cells displaying a key role in the inflammatory process there are monocytes expressing TLR in membrane and that, due to these receptors, are suitable to recognize exogenous (pathogenic) and endogenous "danger signals" and activate intracellular signal pathways regulating the synthesis of inflammatory cytokines. Type and amount of cytokines released from monocytes as a result of "danger signal" recognizing by TLRs determine the quality of the response, thus affecting the migration, the differentiation and the functional state of native immune cells, and regulate the antigen presentation and naive T lymphocyte differentiating destiny into various effector or regulator lineages.

Therefore, both the regulation of the synthesis and the type of cytokines released as response to an inflammatory stimulus represent a key mechanism in inflammation that, if not opportunely controlled, results in tissue damage and onset of chronic inflammatory pathologies.

Among factors released in inflammation site during the inflammatory response there is Nerve Growth Factor (NGF). Basal production of NGF occurring in human tissues increases considerably in the inflamed tissues as result of various chronic and/or autoimmune inflammatory diseases: elevated NGF concentrations seem to be correlated with the inflammation degree and clinical activity in patients with inflammatory chronic diseases as for example multiple sclerosis (MS), rheumatoid arthritis (RA) systemic lupus erythematosus (SLE), Crohn's disease (CD), colitis, psoriasis, atopic dermatitis (for a recent review see ref.2).

NGF local increase appears to be correlated with the occurrence of inflammatory cytokines. In vitro studies have demonstrated that the inflammatory cytokines like Interleukine-1β (IL-1β), Interleukine-6 (IL-6) and Tumour Necrosis Factor-α (TNF-α), induce the synthesis of NGF in various cell types like synoviocytes, fibroblasts, endothelial and glial cells. Also immune cells involved in native immunity have a NGF basal synthesis that increases after the stimulation with specific antigens or cytokines (2). These data, obtained mostly in vitro, demonstrate that the induction of the NGF synthesis could be correlatable to the inflammatory phenomenon degree and have led to the hypothesis of a pro-inflammatory activity of said neurotrophin.

This interpretation is apparently contrasting with reference to in vivo experiments demonstrating that, for inflammatory diseases on animal models, the exogenous administration of NGF, after induction of the disease, reduces the onset and the gravity of clinical symptoms as well as the tissue inflammation on animal models for multiple sclerosis, colitis and UV irradiation.

The intraventricular administration of exogenous NGF, a week after the induction of the autoimmune encephalomyelitis (EAE) delays the onset of the disease in marmoset monkeys, reducing the lesion number and severity. In the brain of NGF treated animals the number and sizes of inflammatory infiltrates are reduced, the demyelination is minimal, the production of IFN-γ is diminished while that of IL-10 is increased (3). This effect of the NGF on the inflammatory response for EAE has been observed also in murine models obtained with various methods for disease induction and wherein the intraperitoneal injections of NGF delay the disease onset, decrease the clinical symptoms and increase the survival of animals (4,5). As an ulterior confirmation of these results, the neutralization of NGF obtained with the treatment with anti-NGF antibodies aggravates the symptoms of EAE and increases the number of cerebral inflammatory lesions (6).

The possibility that NGF can be involved in anti-inflammatory pathway in vivo is further supported by studies on murine models of acute inflammation like colitis induced by trinitrobenzene sulfonic acid (TNBS) and skin irradiation using UVB. For mouse epidermis the UVB radiation results in a NGF increase reaching maximum value after 12 hours (7). If endogenous NGF is neutralized with anti-NGF a contact hypersensitivity response (contact hypersensitivity responses-CHS) is induced that on the contrary normally is inhibited by UVB. In a similar way, the administration of NGF in normal mice before the treatment of the cutis with trinitrochlorobenzene inhibits CHS response (8).

Similar results are obtained in colitis models wherein it is showed that NGF synthesis is maintained elevated until the inflammatory response (9) does not decrease. NGF deprivation, obtained in these models with anti-NGF antibodies, gets meaningfully worse inflammatory phenomenon, determining a worsening of clinical symptoms, an increase of the number of inflammatory lesions characterized moreover by a larger infiltrate of inflammatory cells (10).

In these acute inflammation models, the NGF presence, produced during the inflammatory response, seems to be necessary in order the organism ability to control the inflammatory response to be regulated and anti-inflammatory mechanisms limiting tissue damage to be activated.

NGF mechanism of action and effects of NGF receptor block on the peripheral sensory neurons are characterized enough. In fact, the neutralization of NGF with anti-NGF antibodies or the block of TrkA receptor have been suggested as possible pharmacological treatments for the treatment of variously originating pain. On the contrary, it is unknown which are NGF modulated anti-inflammatory mechanisms, in which way this neurotrophin acts for regulation thereof, the reasons leading to NGF production during the inflammatory response and physiological role thereof, that is it is completely unknown which are the effects of NGF receptor inhibition on immune cells involved in inflammatory response. In fact, in addition to unknown reasons by which the NGF production increases during the inflammation and cellular and molecular mechanisms regulated by NGF during the inflammatory response, further it is unknown whether the expression of TrkA and p75NTR is modulated during the inflammation and which are the signal pathways activated by these two receptors.

TrkA and p75NTR intracellular signals have been investigated almost exclusively in neuronal cells. In neuronal cells the NGF activity is mediated by binding thereof to two membrane receptors: TrkA and p75NTR. TrkA is a 140 kD transmembrane tyrosine kinase that is phosphorylated at tyrosine residues when it binds NGF. Main intracellular signal pathways activated by TrkA are Ras, phosphatidylinositol-3-kinase (PI3-kinase), phospholipase C-γ1 (PLC-γ1) and downstream effectors thereof, including the stimulation through Ras of mitogen-activated protein (MAP) kinase cascade, Akt-dependent PI3-kinase stimulation and PLC-γ1 induced IP-3 and diacyl-glycerol formation, which, in turn, determine the mobilization of intracellular calcium (11). The other NGF receptor, p75NTR, is a 75 kD glycoprotein belonging to the family of TNF receptors. Contrarily to TrkA, p75NTR does not have catalytic intracellular sub-units but binds adapters like sortilin, NOGO, RhoGDI, Traf6 etc., thus activating intracellular signal pathways. Among p75NTR activated signal pathways there are: signal cascade activated by c-Jun kinase, (JNK) NF-kb activation, sphingomyelinase activation, ceramide formation and RhoA activation (11). Intracellular signal pathways activated by NGF through receptors thereof have been studied mostly in neuronal cells and very little data is available about which are TrkA and p75NTR activated signal pathways. The majority of immune cells expresses TrkA (2) while the dendritic ones seem to express mostly p75NTR (12,13). The expression of TrkA is finely regulated within lymphocytes and monocytes and increases after stimulation with inflammatory stimulus (2). Studies carried out on lymphocytes have demonstrated that NGF and TrkA binding induces activation of MAP-kinase pathway (14). On the other hand up to now it is unknown which signal pathways are activated by NGF through TrkA and p75NTR in the immune cells and in which way the activation thereof affects the activity of the immune cells.

Indeed these information is important in order to understand the mechanisms through which NGF regulates the inflammatory response.

Until now, the majority of described NGF biological activities is considered induced by the activation of TrkA and thereto associated signal pathways regulating the activation or inactivation of NGF controlled genes. Until few years ago it was thought that the function of p75NTR, which is prototype protein of TNF receptor superfamily, was to make more specific NGF binding to TrkA and increase the affinity thereof, creating high affinity heterodimeric receptors, i.e. p75NTR-TrkA, Recent studies with structural biochemical analysis on the contrary have demonstrated that TrkA and p75-NTR forms homodimers and not heterodimers with NGF (15), therefore the emerging hypothesis is that the same are signal pathways independently activated by NGF binding with each of two receptors thereof to converge downstream and through adapter molecules (16). The NGF biological activity depends therefore on a convergence of signals activated by each receptor rather than by direct interactions of two receptors at level of the extracellular surface.

It is known that in neurons TrkA and p75NTR control various signal pathways leading to different intracellular responses. For example in nervous system during development NGF can act both as trophic and apoptotic factor depending on the prevalence of the signal pathways activated by TrkA (survival) or p75NTR (apoptosis). There are not many studies out of the nervous system demonstrating the different responses NGF can evoke through the selective stimulation of one or other receptor and these information is completely absent for immune cells and particularly for monocytes exerting a key role in inflammation. The only study wherein a particular role is assumed for p75NTR is by Micera et al., 2007, wherein it is demonstrated that in fibroblasts (connective, not immune, tissue cells), obtained from conjunctiva of allergic keratoconjunctivitis patients, the block of p75NTR results in an increase of the secretion of connective tissue protein (17).

In addition, the study by Barthel et al., 2009 shows that both TrkA and p75NTR are overexpressed in inflamed tissues (39).However, how TrkA and p75NTR are modulated during the inflammatory response, activated signal pathways and how these can interfere with the activation of TLRs and synthesis of inflammatory cytokines is all currently unknown information.

Contrarily to the prevailing hypothesis according to which NGF is an inflammatory factor, the inventors of the present invention have based their studies on diametrically opposite considerations. The inventors have, in fact, assumed that the increased synthesis of NGF, characterizing the sites of inflammation of animal models for acute inflammation and patients with chronic inflammation or autoimmune diseases, is an endogenous mechanism activated to modulate the inflammatory response and limit tissue damages. The working hypothesis has been, therefore, that the relative expression of two NGF receptors is the critical factor determining the ultimate effect of NGF on inflammatory response. As it is currently known only for nervous cells (11) and fibroblasts (17), two NGF receptors, i.e. TrkA and p75NTR, can regulate different signal pathways and give rise to apparently contrasting NGF effects (i.e survival/apoptosis). The general purpose of the present study has been therefore to acquire new information on the role of NGF and TrkA and p75NTR receptors in the inflammation and how the inhibition of either receptors can modulate the inflammatory response.

The inventors of the present invention now have found, by means of studies on human monocytes, that the binding of NGF to TrkA and p75NTR activates independently to each other signal pathways and suitable on turn to modulate the intracellular signals of inflammatory stimuli activated TLRs, controlling in fact the production of inflammatory cytokines. The data demonstrates for the first time that NGF can act on the same cell in opposite ways depending on the prevalence of one of the two receptors. In particular, the inventors, by studies on monocytes, have demonstrated that:
- TrkA receptor has an anti-inflammatory role;
- p75NTR receptor has a pro-inflammatory role.

The data demonstrates, moreover, that in patients affected by rheumatoid arthritis or juvenile idiopathic arthritis, typical chronic inflammatory diseases, there is an anomalous p75NTR/TrkA ratio, p75NTR being higher thus favouring pro-inflammatory p75NTR mediated NGF signal. Said new information obtained by studies about intracellular pathways activated by NGF binding to TrkA and p75NTR, on the effects of the activation of either NGF receptor, TLR activation and cytokines synthesis allowed p75NTR receptor to be identified as a new target for the therapy of chronic inflammatory diseases.

In vitro obtained data using human monocytes and culture conditions modulating the expression of TrkA and p75NTR receptors changing relative ratio thereof, along with the use of neutralizing antibodies, p75NTR pharmacological inhibitors and some intracellular intermediates, allowed to demonstrate for the first time that:
a) TrkA and p75NTR controls different intracellular pathways;
b) NGF acts through receptors thereof in order to regulate TLR activated signal pathways and modulate the synthesis of cytokines in the monocytes which are cells displaying a key role in inflammation and critical link between native and adaptive immunity;
c) the blocking of a receptor in fact strengths the activity of NGF through the other receptor and, since the controlled signal pathways are different for said two receptors, the NGF activity on cytokine synthesis turns out diametrically opposite;
d) the activity of p75 mediated NGF is pro-inflammatory and p75NTR block with neutralizing antibodies or pharmacological inhibitors results in a reduction of inflammatory cytokines and prevalence of the anti-inflammatory pathways which on the contrary are modulated by TrkA;
e) mono-nucleated cells from patients affected by rheumatoid arthritis (RA) and Juvenile Idiopathic Arthritis (JIA) have a p75NTR/TrkA expression ratio inverted than cells from healthy individuals, with prevalence of p75NTR in patients with chronic inflammation while normally the mainly expressed receptor in healthy subjects is TrkA;
f) synoviocytes from RA patients maintain this higher p75NTR expression associated with a spontaneous production of inflammatory cytokines that is reduced by inhibition of p75NTR receptor.

In conclusion, existing data have pointed out a new pathogenic mechanism displaying an abnormal expression of p75NTR in patients with chronic inflammation that, associated to increased production of NGF, results in an disequilibrium of pro- and anti-inflammatory (18) pathways normally activated during the inflammatory response. The greater activation of p75NTR determines an enhancement of pro-inflammatory ways of signal activated by TLRs (i.e. NF-kb) and a greater production of inflammatory cytokines. On the contrary in normal conditions NGF signals through p75NTR, but above all through TrkA whose expression is enormously induced by the inflammatory stimuli in mononucleated cells of healthy subjects. This altered expression of p75NTR in patients with chronic inflammation could contribute to the dysregulation of signal pathways linked to Toll-like receptors resulting in an increase of the synthesis of inflammatory cytokines and chemokines which, in association with alterations in endogenous anti-inflammatory mechanisms, could therefore contribute to the chronic inflammation.

The identification of this new pathogenic mechanism and data obtained on the effects of p75NTR inhibition on the production of inflammatory cytokines in models in vitro and ex vivo experiments suggest p75NTR to be a new therapeutic target in chronic inflammation.

In the light of herein described results, inhibitors (antagonist) of p75NTR receptor and activators (agonists) of TrkA receptor represent useful molecules for modulating the inflammatory response in chronic inflammatory diseases wherein TrkA/p75 receptor ratio is altered.

Until now the agonists and antagonists of NGF receptors are conceived in order to regulate neuronal alterations (pain therapy, neuropathies and like) or neuron survival (Alzheimer's disease) being based on known and well described mechanisms involving NGF within neurons. The article of Petersen et al., 1998, for instance, concerns an in vitro study curried out on sensory neurons and suggests a role of NGF in the activation of pain via p75NTR receptor. Particularly, it concerns an in vitro study showing that NGF increases the expression of bradykinin binding sites on neurons via p75NTR, bradykinin being one of the most powerful endogenous pain-producing substances that is released following tissue injury. The authors envisage that the interference with NGF-p75NTR signaling mechanisms may reveal new treatment strategies for chronic inflammatory pain states.

Currently, two monoclonal antibodies, NGF or TrkA respectively neutralizing, are in initial clinical experimentation for pain therapy in neuropathies.

In addition there are reported in scientific literature some compounds synthesized and used in order to inhibit the NGF and p75NTR or TrkA binding in vivo and in vitro experiments (19-37). The use thereof is finalized to neurodegenerative diseases. Currently the following compounds have been described:
- Neutralizing monoclonal antibodies and chimeric antibodies or soluble receptors, as those already used in experiments carried out in our laboratory and like humanized monoclonal antibody MNAC13 against TrkA, now BXL1H5 (Bioxell); monoclonal antibody blocking NGF-TrkA binding, tanezumab, previously known as RN624 (Pfizer); soluble receptor TrkAD5 (soluble TrkA-NGF binding domain, previously known as REN 1820); 5C3 TrkA agonist (37) monoclonal antibody; anti-p75NTR ME20.4 neutralizing monoclonal antibody; p75NTR neutralizing MLR-1, MLR2, MLR3 monoclonal antibodies; anti-p75NTR human clone HB-8737 antibody, anti-p75NTR neutralizing polyclonal antibody (REX).
- p75NTR binding NGF analogous cyclic monomeric and dimeric compounds, as NGF C30-35, or TrkA binding, as NGF C92-97, preventing NGF binding (19-23) or acting as TrkA agonists as D3 (25) or mutated recombinant NGF as NGF KKE/7-84-103 (26,27).
- PD90780 compound which binds NGF preventing the binding thereof to p75NTR partially influencing also the binding with TrkA (28-30); gambogic amide, small molecule mimicking NGF activity and representing a new selective TrkA agonist (31).
- compounds belonging to LM11A family and among these LM11A-24 and LM11A-31 derived from caffeine and isoleucine, respectively. Said compounds consist of not peptide small molecules binding p75NTR in NGF specific binding site inhibiting selectively the ability of p75NTR to bind NGF but not interfering in any way NGF and TrkA binding (33-36);
- low molecular weight not peptide molecules comprising 3-aza-bicylo [3.2.1] octane and monomeric and dimeric derivatives thereof binding TrkA but not p75NTR and acting as human neurotrophin agonists (Patent US 7625892).

Above reported data demonstrate that p75NTR antagonists, being neutralizing monoclonal antibodies, chimeras or antagonists like LM11A not peptide molecules, alone or in association with NGF or TrkA agonists, activate intracellular pathways inhibiting the inflammatory response, thus resulting in a reduction of production of inflammatory cytokines and an increase of production of anti-inflammatory cytokines.

Overlapping results have been obtained by blocking p75NTR with various substances using different inhibition mechanisms:
a) neutralizing antibodies, which through the receptor binding prevent NGF and p75NTR binding but do not interfere with NGF and TrkA binding
b) soluble p75NTR receptor (chimeric recombinant molecule consisting of amino acid sequence of the extracellular region of human p75NTR receptor fused to a Fc region of human IgG) that binds occurring soluble NGF recognizing NGF specific binding site with p75NTR which is different than TrkA.
c) LM11A-24 LM11A-28, LM11A-31 type molecules, derived from caffeine or isoleucine that are non-peptidyl molecules, mimetics of neurotrophin loop 1 domain and therefore occupy in p75NTR NGF binding site preventing NGF from interaction with p75NTR that can therefore binds only TrkA.

Independently on used inhibition type, the effects of p75NTR blocking, as below illustrated, results always in a reduction of the synthesis of inflammatory cytokines and an increase of the anti-inflammatory cytokines, and demonstrate in an unambiguous way the therapeutic potential of p75NTR block.

It is therefore a specific object of the present invention a compound (or more compounds) selected from p75NTR receptor inhibitors (or antagonists), alone or in association with at least one compound selected from TrkA receptor activators (or agonists), or a compound selected from TrkA receptor activators, for use in anti-inflammatory treatment of chronic inflammatory diseases of auto-inflammatory or autoimmune origin, with exclusion of allergic origin diseases, in patients wherein the expression ratio TrkA receptor/p75NTR receptor is lower than 1 in inflamed tissues or cells of biological fluids; wherein the inhibiting compounds (antagonists) of p75NTR receptor can be selected from the group consisting of p75NTR binding, polyclonal and monoclonal, monospecific or bispecific antibodies, chimeric molecules whose structure includes soluble p75NTR, p75NTR binding cyclic monomeric and dimeric NGF analogous compounds and wherein the activators (agonists) of TrkA receptor can be selected from the group consisting of TrkA binding polyclonal and monoclonal antibodies as 5C3 monoclonal antibody (49), cyclic monomeric and dimeric NGF analogous compounds as NGF C92-97 or recombinant mutated NGF, for example loop 1 mutated and thus not p75NTR receptor binding, as NGFKKE/7-84-103, D3 peptide, gambogic amide, and 3-aza-bicyclo [3, 2, 1] octane derivatives (US7,625,892).

The p75NTR binding monoclonal antibodies can be selected from the group consisting of monoclonal neutralizing anti-nerve growth factor receptor p75 human antibody, clone ME20.4 from Chemicon; MLR-1, MLR2, MLR3 monoclonal antibodies, anti-p75NTR human clone HB-8737 (ATCC) antibody. Soluble p75NTR receptor, that it is a chimerical molecule consisting of amino acid sequence of the extracellular region of human p75NTR receptor fused to human IgG Fc region can be, for example, human NGFR/TNFRSF16/Fc recombinant chimera from R&D Systems.

The p75NTR binding polyclonal antibody can be for example anti-p75NTR (REX) neutralizing polyclonal antibody or rabbit 9651 and 9650 polyclonal antibodies recognizing p75NTR extracellular domain. p75NTR binding cyclic monomeric and dimeric NGF analogous compounds can be selected among NGF C30-35, PD90780 compound, LM11A compounds.

LM11A compounds can be selected from the group consisting of LM11A-24 caffeine derivatives or LM11A-31 isoleucine derivatives. The chronic inflammatory diseases that can be treated according to the present invention are selected from the group consisting of rheumatoid arthritis, juvenile idiopathic arthritis, intestinal chronic inflammatory diseases (Crohn Disease, ulcerous colitis) ankylosing spondylitis, psoriasis, multiple sclerosis, Lupus erythematosus, scleroderma, Syndrome of Sjogren. Allergic origin diseases are not included.

It is a further object of the present invention a combination of at least one inhibiting compound of p75NTR receptor with at least one activator of TrkA receptor, such as NGF, for the separated or sequential use in anti-inflammatory treatment of chronic inflammatory diseases of auto-inflammatory or autoimmune origin, with exclusion of allergic origin diseases, in patients wherein the expression ratio TrkA receptor/p75NTR receptor is lower than 1 in inflamed tissues or cells of biological fluids; wherein the inhibiting compounds (antagonists) of p75NTR receptor can be selected from the group consisting of p75NTR binding, polyclonal and monoclonal, monospecific or bispecific antibodies, chimeric molecules whose structure includes soluble p75NTR, p75NTR binding cyclic monomeric and dimeric NGF analogous compounds and wherein the activators (agonists) of TrkA receptor can be selected from the group consisting of TrkA binding polyclonal and monoclonal antibodies, cyclic monomeric and dimeric NGF analogous compounds or recombinant mutated NGF.
The chronic inflammatory diseases that can be treated according to the present invention are selected from the group consisting of rheumatoid arthritis, juvenile idiopathic arthritis, intestinal chronic inflammatory diseases (Crohn Disease, ulcerous colitis), ankylosing spondylitis, psoriasis, multiple sclerosis, Lupus erythematosus, scleroderma, Syndrome of Sjogren.

In addition, based on experimental data obtained by the inventors of the present invention, it is possible to assume that, for patients affected by chronic inflammatory diseases of auto-inflammatory or autoimmune origin, with exclusion of allergic origin diseases, an expression ratio of TrkA and p75NTR receptors lower than 1 can be predictive of response to treatment with p75NTR inhibitors alone or in association with TrkA agonists or currently used therapies. Therefore, the evaluation of said ratio could be employed for an in vitro diagnostic method in order to estimate the possibility of response to the therapy. In particular, the method could be based on the quantitative determination of mRNA levels for p75NTR and TrkA estimated in inflamed tissue biopsies as, for example synovium, skin, kidney, intestine, or cells obtained from biological fluids, for example blood, urines, synovial liquid, cephalorachidian liquor, saliva.

A further object of the present invention is an in vitro or ex vivo screening method in order to estimate the anti-inflammatory effect of inhibitors of p75NTR receptor in chronic inflammatory diseases of auto-inflammatory or autoimmune origin, with exclusion of allergic origin diseases, directly on biological samples, as for example isolated tissues or mononucleated cells of a patient, said method comprising or consisting of the following steps: a) contacting isolated tissues or mononucleated cells of a patient affected by chronic inflammatory disease with said inhibitor (antagonist); b) measuring the levels of pro-inflammatory cytokines, as for example IL-1, IL-6 and TNF, released spontaneously or after stimulation with TLR ligands, wherein said chronic inflammatory diseases are selected from the group consisting of rheumatoid arthritis, juvenile idiopathic arthritis, intestinal chronic inflammatory diseases (Crohn Disease, ulcerous colitis), ankylosing spondylitis, psoriasis, multiple sclerosis, Lupus erythematosus, scleroderma, Syndrome of Sjogren. The model uses monocytes or mononucleated cells from peripheral blood or sinoviocytes from sinovium or fibroblasts from cutis or intestinal epithelial cells and evaluates the possible decrease of production of inflammatory cytokines and induction of anti-inflammatory cytokines before and after treating cells with p75NTR inhibitors and/or TrkA activators.

The present invention now will be described by an illustrative way according to preferred embodiments thereof, with particular reference to attached drawings wherein:

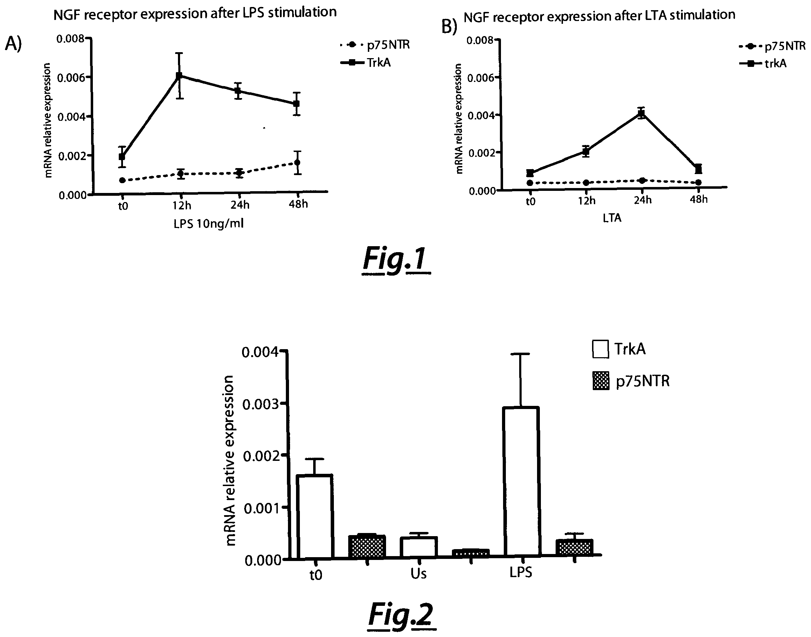

Figure 1: human monocytes stimulated with TLR4 (A) and TLR2 (B) ligands increase the expression of TrkA over following hours while the expression of p75NTR is more stable over the time.
Figure 2: just purified peripheral blood monocytes (t0) express both TrkA and p75NTR, TrkA being expressed in larger amounts. After 24 hours without stimulation (Us) the expression of TrkA and p75, as estimated by real time-PCR, decreases while TLR stimulation with LPS induces a meaningful increase of TrkA expression (LPS).

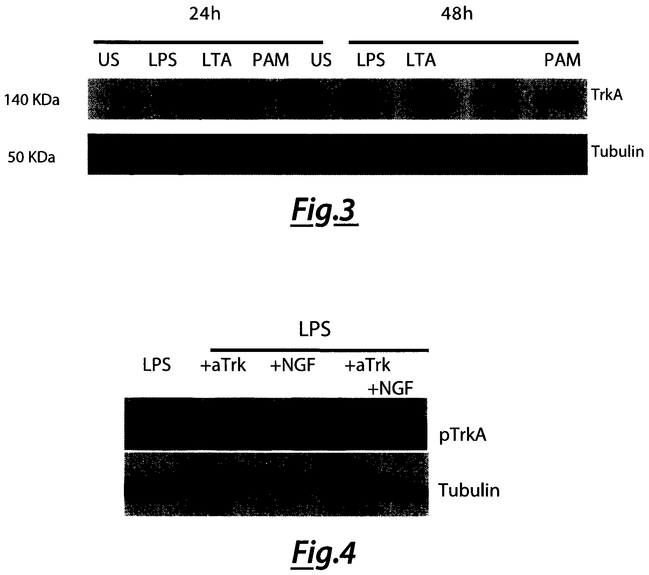

Figure 3: Western blot of TrkA after 24 and 48 hours of stimulation with TLR ligands. While under not stimulating conditions (Us) there is a very low expression of TrkA, after TLR4 and TLR2 activation with LPS and LTA or PMA, respectively, an increase of TrkA after 24 hours and lasting also after 48 hours is observed.
Figure 4: phosphorylation of TrkA receptors in monocytes after NGF binding. In LPS stimulated monocytes the addition of exogenous NGF induces an auto-phosphorylation of receptor that is effectively inhibited by anti TrkA antibodies.

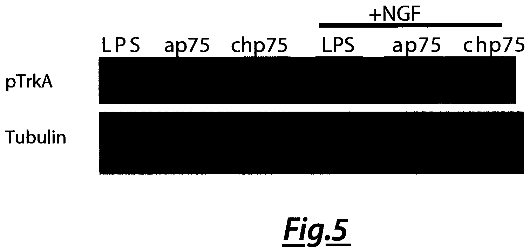

Figure 5: The addition of inhibitor preventing NGF from p75NTR binding does not change the level of TrkA phosphorylation induced by the addition (administration) of NGF to LPS activated monocytes. Although p75NTR cannot bind NGF, the NGF ability to phosphorylate TrkA is unaffected.

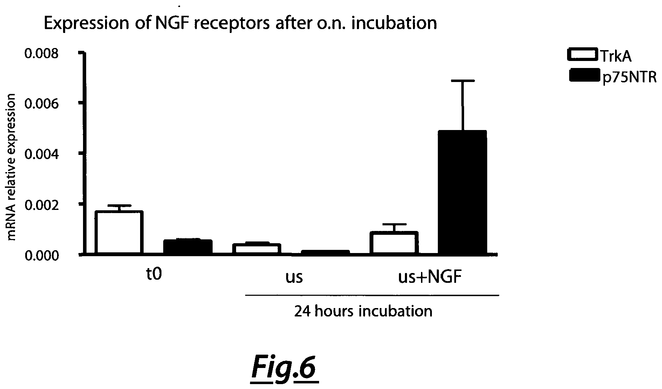

Figure 6: Expression of NGF receptors after 24 hour culture. The levels of TrkA and p75NTR expression are analyzed in just purified monocytes (t0), after 24 hour incubation in stimuli free or NGF added media.

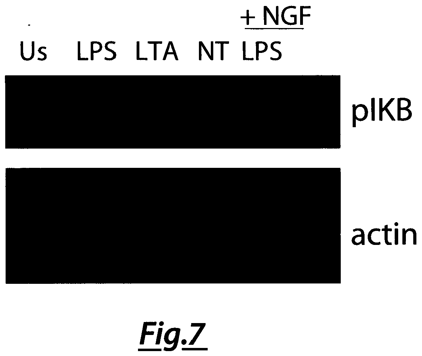

Figure 7: In 16 hour NGF treated cells and having a greater expression of p75NTR, the stimulation with LPS induces a greater phosphorylation of IkB than cells treated only with LPS. Not stimulated cells (us) do not show IkB phosphorylation.

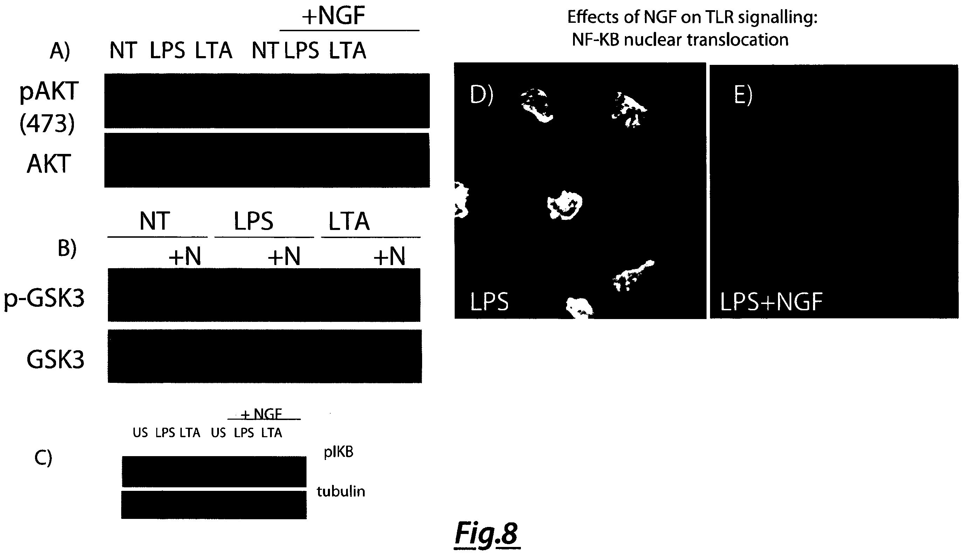

Figure 8: NGF influences TLR activated intracellular pathways. When TrkA is expressed in higher amount, in the cells stimulated with LPS or LTA the NGF addition increases activating AKT phosphorylation (A) and also affects GSK3 (B) inhibiting phosphorylation. In addition to this activation of anti-inflammatory pathways, NGF affects consequently IkB phosphorylation reducing the same (C) and thus also reducing the NF-kb translocation into the nucleus (D-E). In figures D-E green fluorescence is specific for NF-kb p65, blue colour represents the nucleus and white colour indicates nucleus translated NF-kb p65.

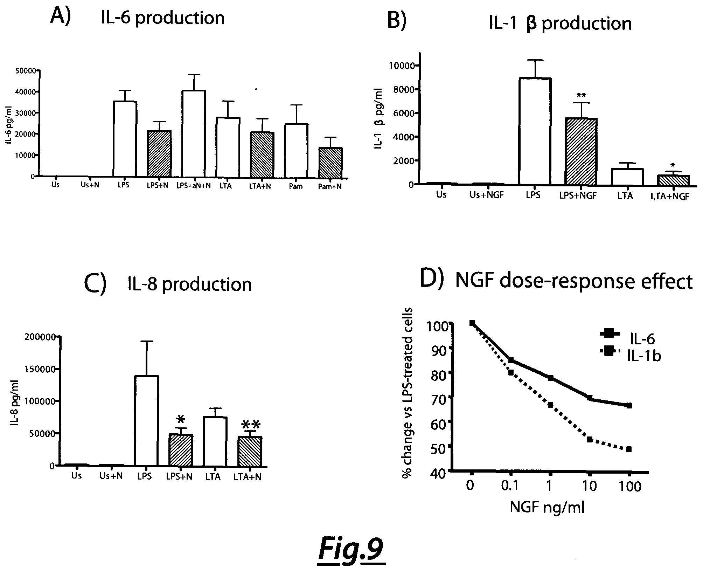

Figure 9: NGF reduces the production of inflammatory cytokines in monocytes expressing elevated levels of TrkA. In cells activated with LPS or LTA inducing an increase of expression of TrkA, the NGF addition decreases the production of IL-6 (A), IL-1 b (B) and IL-8 (C) in the monocytes. In not stimulated (US) cells, NGF does not induce cytokines synthesis as such. The inhibition effect thereof on release of inflammatory cytokines is dose dependent (D).

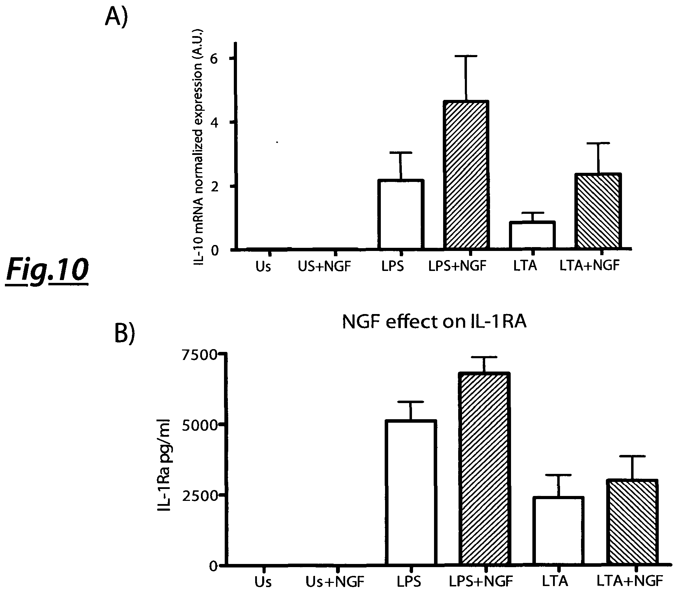

Figure 10: NGF increases the production of anti-inflammatory cytokines in monocytes expressing elevated levels of TrkA. In cells activated with LPS or LTA inducing an increase of expression of TrkA, the NGF addition increases the synthesis of IL-10 (A), IL-1ra (B) in monocytes.

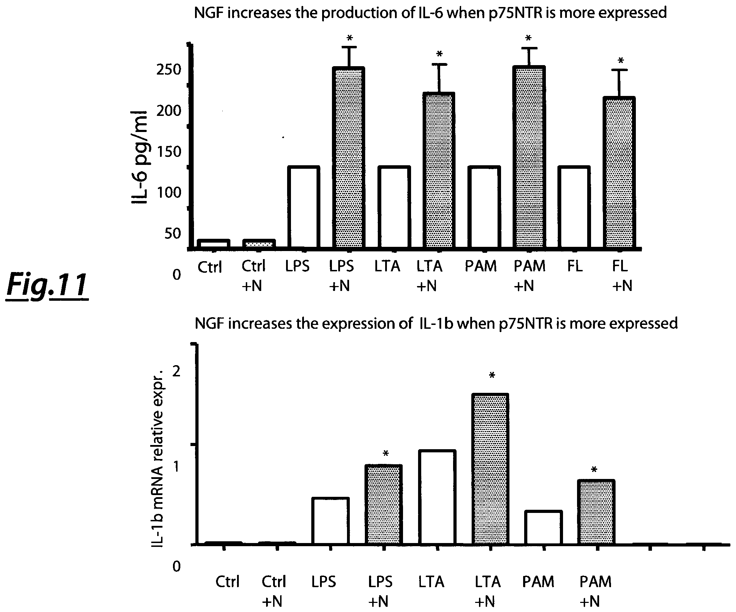

Figure 11: NGF increases the production of inflammatory cytokines in monocytes expressing elevated levels of p75NTR. In cells activated with LPS, LTA or PAM in monocytes having an elevated expression of p75 NTR, the addition of NGF increases the production of IL-6 (A), IL-1 b (B) in monocytes. In not stimulated cells (US), NGF does not induce cytokines synthesis as such.

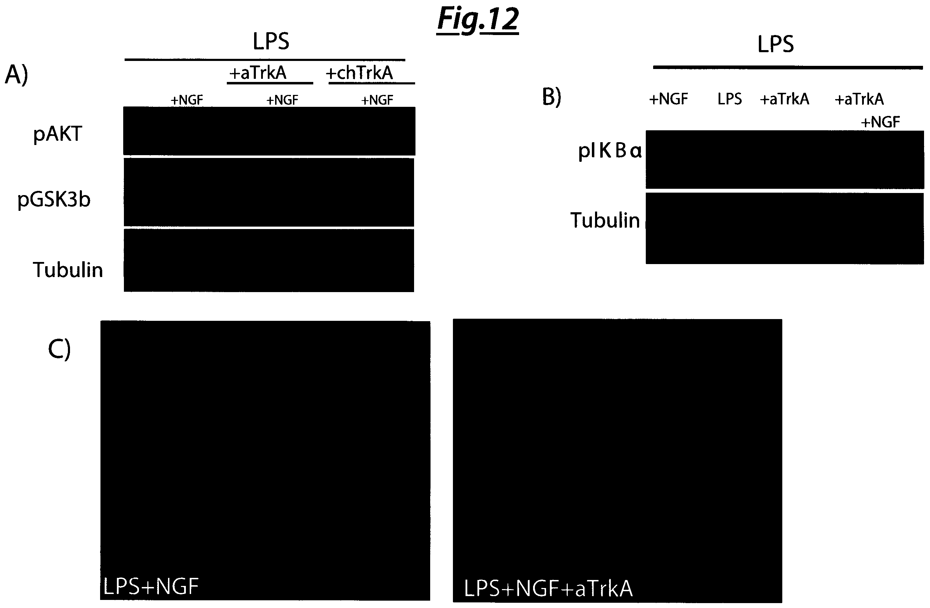

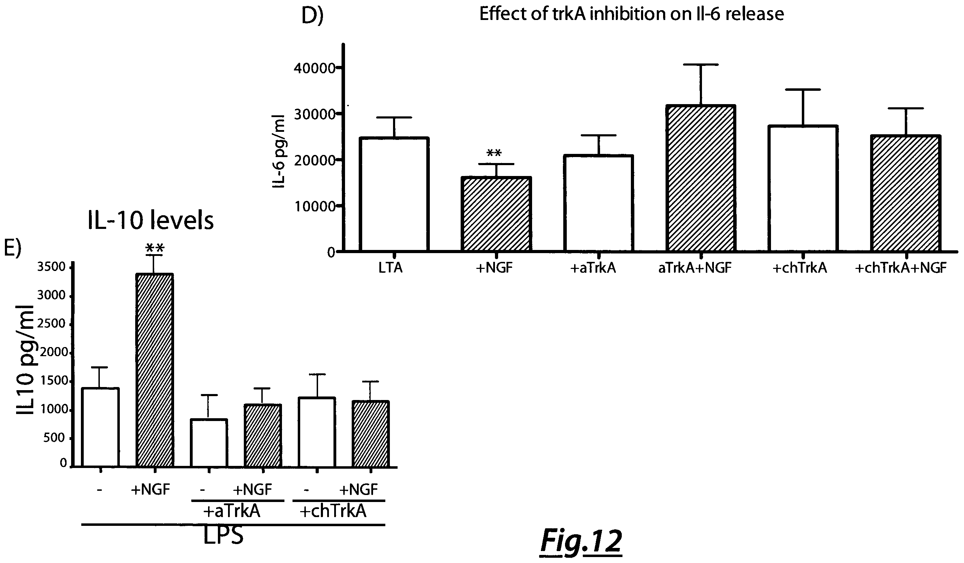

Figure 12: The inhibition of TrkA blocks the anti-inflammatory activity of NGF. In LPS activated cells the TrkA inhibition, both with neutralizing antibodies (aTrkA) and soluble receptor (chTrkA), results in a decrease of AKT phosphorylation and GSK3 inhibiting phosphorylation (Fig. 12a), increase of IkB phosphorylation (Fig 12b), nuclear translocation of NF-kb (Fig 12c), IL-6 production (Fig 12D) and IL-10 reduction (Fig 12E). In Figure C green fluorescence is specific for NF-kb p65, blue colour represents the nucleus and white colour indicates nucleus trans-located NF-kb p65.

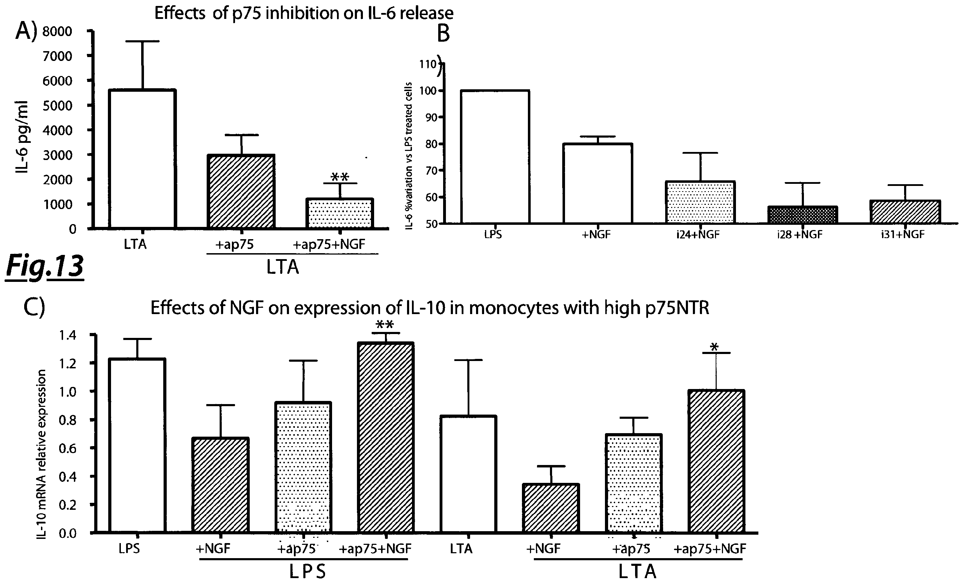

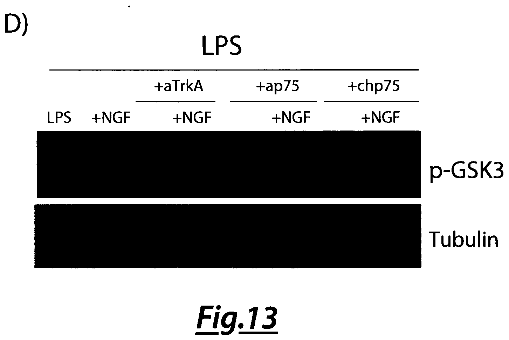

Figure 13: The inhibition of p75NTR reduces the inflammatory pathway induced by TLR. Monoclonal antibody (ap75) or p75NTR chimera (chp75) or mimicking LM11A (i24,i28, i31) molecules decrease the synthesis of IL-6 in monocytes activated with LPS (Fig. 13 a-b), increase the IL-10 synthesis (Fig. 13c). This regulation occurs through the activation of PI3K/AKT pathway and inhibiting GSK3 phosphorylation (Figure 13d).

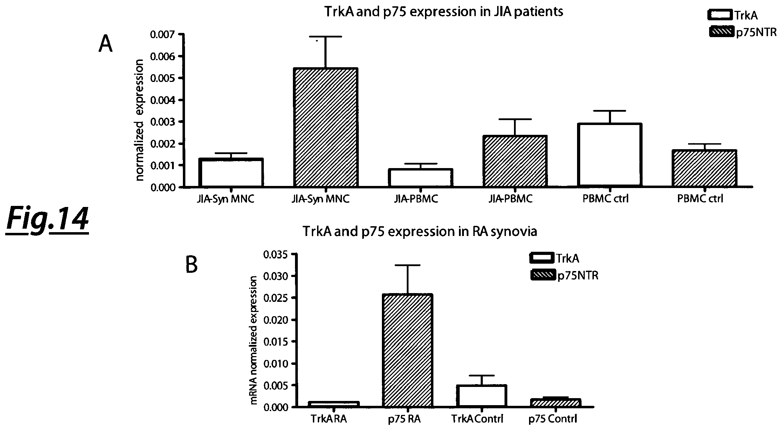

Figure 14: Expression of TrkA in patients affected by chronic inflammation. The expression of p75NTR is very increased in mononuclear cells obtained from synovial liquids (JIA Syn-MNC) and peripheral blood (JIA-PBMC) from patients affected by Juvenile Idiopathic Arthritis while TrkA expression is meaningfully reduced compared to healthy controls (ctrl) (Fig 14a). An increase still more apparent of p75NTR and a decrease of TrkA characterize synovia of the patients affected by rheumatoid arthritis (TrkA RA; p75 RA) (Fig 14b).

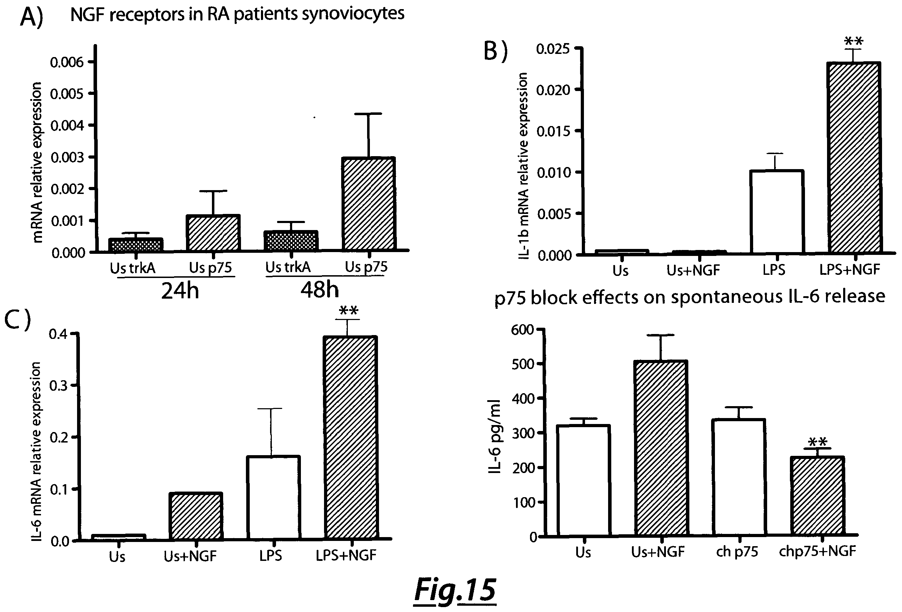

Figure 15: Human synoviocytes from patients suffering by rheumatoid arthritis ex vivo reduces the production of inflammatory cytokines after treatment with p75NTR inhibitors. The expression of p75NTR is high and greater than TrkA in synoviocytes obtained from synovia of patients affected by rheumatoid arthritis (Fig15a). The addition in vitro of p75NTR inhibitor reduces the spontaneous synthesis of IL-6 in cells of patients (Fig15b).

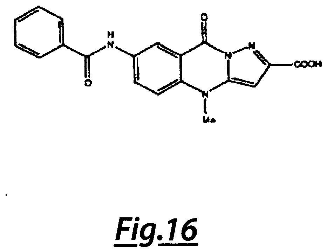

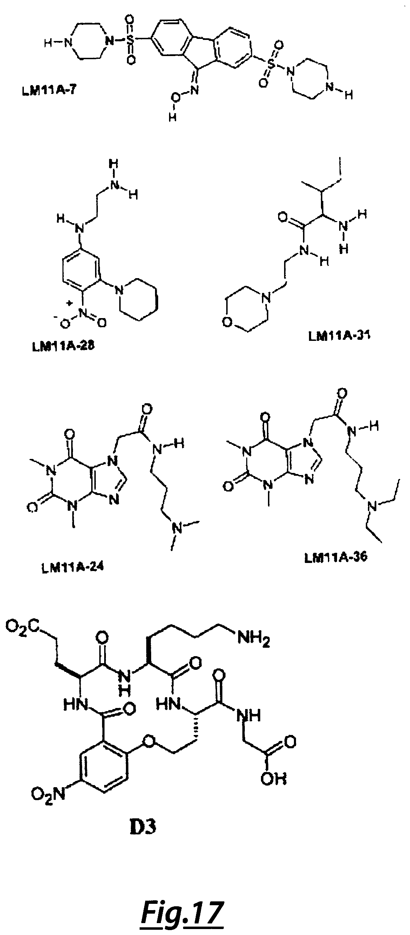

Figure 16 shows the formula of PD90780 compound.
Figure 17 shows formulas of LM11A-7, LM11A-28, LM11A-31, LM11A-24, LM11A-36 and D3 compounds.

### Example 1: Study of the relation between expression of p75NTR and TrkA receptors and chronic inflammatory disease

### METHODS

### Cell Cultures

In this study human monocytes obtained from buffy coats after separation on Ficoll-Hypaque gradient (Pharmacia, Uppsala, Sweden), separation on Percoll discontinuous gradient (Pharmacia, Uppsala, Sweden) as described by Sallusto and Lanzavecchia (1994) and successive negative immunomagnetic selection (Miltenyi Biotec, Germany) have been used. The cell purity is estimated by FACS analysis and CD14 positive monocyte fraction was >95%. Cells have been plated at concentration of 2x10⁶/ml in Falcon 96 or 12 well plates and cultured at 37°C in wet atmosphere at 5% CO₂ and culture medium consisting of RPMI 1640 (Biowhittaker, Walkersville, MD) containing 50 Unit/ml penicillin, 50 µg/ml streptomycin, 2mM L-glutamine (all from Gibco-BRL Life Technology, Gaithersburg MD) and 5% inactivated foetal bovine serum (FBS) (HyClone Labs, Logan, Utah). In order to study the NGF effects when the expression of TrkA or p75NTR on the activation of Toll-like receptors is prevailing and the synthesis of inflammatory cytokines in monocytes, two culture conditions allowing the expression of NGF receptors to be modified are identified. Under A culture condition an increased expression of TrkA and anti-inflammatory effect of NGF in the presence of inflammatory stimulus and activation of the TLR occur. On the other hand under B culture condition an increase of the expression of p75NTR occurs, and in the presence of inflammatory stimulus and TLRs activation a NGF pro-inflammatory effect is observed.
A) Condition: human purified monocytes have been stimulated with TLRs ligands (10 ng/ml LPS, 2µg/ml LTA, 100ng/ml PAM) and at the same time NGF at concentrations from 0.1 to 100ng/ml is added. After stimulation, the conditioned media and cells are collected at different stimulation times from 3 hours to 5 days.
B) Condition: human purified monocytes are pre-incubated with 0.1, 1, 10 and 100 ng/ml NGF for 16 hours and successively stimulated with the following TLRs ligands: 10 ng/ml LPS, 2µg/ml LTA, 100ng/ml PAM. Conditioned media and cells are collected at different stimulation times from 3 hours to 5 days. In the receptor inhibition experiments TrkA or p75NTR receptor neutralizing antibodies at different concentrations from 0.3 to 5 mg/ml are used; chimerical molecules consisting of extracellular domain of TrkA or p75NTR human receptor fused to a Fc region of human IgG acting as soluble receptor at different concentrations from 0,1 to 5 µg/ml; LM11A mimetic molecules binding p75NTR in binding site of NGF loop 1 with a greater affinity and at different concentrations from 0.1 to 100 nM have been used.

### Cytokine measurement by ELISA

Conditioned media have been collected 18 hours after stimulation with TLR ligands, stored at -70°C and then analyzed for determination of various cytokine concentrations. IL-6, IL-1ra, IL-1β, IL-8, TNF-α and IL-10 human cytokines have been determined in conditioned media using ELISA Duoset kits supplied by R&D Systems, Minneapolis, MN, following the manufacturer instructions. ELISA sensitivity was 5.47 pg/ml for IL-6, 39.06 pg/ml for IL-1ra pg/ml, 7.8 pg/ml for IL-1β, 31.25 pg/ml for IL-8, 7.8 pg/ml for TNF-α, and 15.62 pg/ml for IL-10, respectively.

### RNA extraction and PCR Real-Time analysis

Total RNA has been extracted from 2x10⁶ monocytes for each type of treatment and at various incubation times using RNeasy® mini kit (Qiagen GmbH, Germany), and following the manufacturer instructions. The concentration and purity of extracted RNA are measured spectrophotometrically and 1mg of total RNA for each sample has been used for cDNA synthesis using Superscript VILO cDNA synthesis Kit (Invitrogen, Carlsbad, CA).

### PCR Real-Time Analysis

Real-Time polymerase chain reactions (PCR) for analysis of TrkA, p75-NTR, TLR2, TLR4, IL-6, IL-1 and IL-10 expression have been carried out with ABI PRISM™ 7700 Sequence Detector (Applied Biosystems, Foster City, CA), using universal Taqman Master Mix (Applied Biosystems, Foster City, CA). The expressions of TrkA, p75-NTR, IL-6, IL-1 and IL-10 have been measured using Assay on Demand reagents from Applied Biosystems (p75-NTR Hs00182120_m1; IL-6 Hs00985639_m1; IL-1β Hs00174097_m1; IL-10 Hs00961622_m1).

In all the experiments each PCR reaction has been carried out in duplicate. All the obtained expression values for each gene have been normalized with reference to housekeeping gene, namely in these experiments β-actin. (TaqMan® Endogenous Control human β - actin 4326315E, Applied Biosystems, Foster City, CA). The relative quantification has been carried out using C_{T} comparative method (expression levels have been normalized with reference to housekeeping gene expression and calculated as 2^ ΔCt) and obtained results are expressed in arbitrary units (AU).

### Western Blot

The monocytes have been lysed using modified RIPA buffer containing: 50 mM Tris-HCI, pH 7.4; 1% NP-40; 0.1% SDS; 0.25% Nadeoxycholate; 150 mM NaCl; 1 mM EDTA; 1 mM EGTA; 1 mM PMSF; 1 µg/ml each of aprotinin, leupeptin and pepstatin; and 25 mM NaF (all from Sigma). After resolution with SDS-PAGE, the proteins are transferred on nitrocellulose membranes (Amersham Life Sciences, Little Chalfont, UK) and incubated with specific antibodies using standard procedures. Nitrocellulose bound antibodies have been detected by chemiluminescence method with ECL (Amersham Life Sciences, Little Chalfont, UK). In these experiments polyclonal antibodies against TrkA, phospho TrkA, Akt, phospho Akt (Ser 473), GSK3 and phospho GSK3, all from Cell Signaling (Cell Signaling Technology, Danvers, BUT) have been used; monoclonal and polyclonal antibodies against p65 NF-κB, IκBα were from Saint Cruz Biotechnology (Saint Cruz, CA); monoclonal antibodies against tubulin were from Sigma (Saint Louis, MO).

### NFkB Nuclear Translocation

Monocytes have been incubated with TLRs ligands, with or without addition of NGF and inhibitors of TrkA and p75-NTR receptors for 30 minutes. The cells have been fixed for 20 minutes in 4% formaldehyde in PBS, made permeable in cold methanol for 5 minutes, followed by 20 minutes incubation with PBS 2% BSA to block nonspecific sites. The confocal study has been carried out using confocal laser Olympus microscope. Sequential mode acquisition technology allows the emissions from various fluorophores to be differentiated (blue, green, red, infrared emitting). The quantification of NF-κB nuclear intensity has been performed using image analysis program FVAS2.1 from Olympus. DRAQ5 dye (Biostatus) labelled nuclei have been selected and NF-κB fluorescence average intensity and nucleus area have been quantified. At least 3 different fields for each condition have been acquired using 60x immersion objective and 50-100 cells quantified for every condition.

### Patient sample analysis

21 synovial liquid and 14 peripheral blood samples from 27 patients with juvenile idiopathic arthritis have been analyzed. Patient samples have been obtained with informed consent within a plan for the expression evaluation of genes correlated to juvenile arthritis course approved by OPBG ethical Committee on 26.03.2010 according to reference n°81/10.

Peripheral blood samples from 16 healthy individuals have been used as controls. In addition 6 samples of synovial tissue from adult patients affected by rheumatoid factor positive rheumatoid arthritis have been analyzed.

Total RNA has been extracted from synovial tissue samples or mononucleated cells obtained after separations on Ficoll gradient (Pharmacia) from synovial liquids or patient blood using Trizol and following the manufacturer instructions. The concentration and purity of extracted RNA have been measured spectrophotometrically and 1µg of total RNA for each sample has been used for cDNA synthesis using Superscript VILO cDNA synthesis Kit (Invitrogen, Carlsbad, CA).

In ex vivo experiments mononucleated cells from juvenile idiopathic arthritis patients or synoviocytes from rheumatoid arthritis patients incubated for 24 hours with p75NTR inhibitors with or without addition of NGF in 10% FCS DMEM or RPMI media have been used. The spontaneous release of inflammatory cytokines or response to TLRs ligands then have been estimated in conditioned media by ELISA as previously described.

### RESULTS

The aim of the study was to understand in which way the increased local production of NGF, during the inflammatory process, can affect the synthesis of cytokines by monocytes/macrophages (M/M) and the role performed by NGF receptors in this response. Obtained data have demonstrated the occurrence of two different mechanisms by which NGF acts on inflammatory response of monocytes and depending on the prevalence of either NGF receptors. During the study two culture conditions allowing the expression of NGF receptors to be modified according to prevalence of TrkA or 75NTR have been identified. Under A culture condition an increased expression of TrkA and an anti-inflammatory effect of NGF in the presence of inflammatory stimulus and activation of the TLR occur. On the other hand under B culture condition an increase of the expression of p75NTR occurs, and in the presence of inflammatory stimulus and TLRs activation a NGF pro-inflammatory effect is observed.

*Analyses of the expression of NGF receptors and associated signal pathways.*

Since the biological activity of NGF is mediated by the binding thereof to specific receptors, we have estimated the expression levels of p75NTR and TrkA in monocytes under above reported two different culture conditions and administration routes. In monocytes a basal TrkA expression exists that is modulated by TLRs activation with specific ligands (Fig1).

TrkA increases following TLRs activation both as a messenger and protein (Fig. 2 and 3). On the contrary the expression of p75NTR remains quite stable and it does not increase in meaningful way after TLRs activation (Fig. 1 and 2). TrkA receptors, induced in the monocytes by TLRs activation, are functionally active and when exogenous NGF is added then autophosphorylation of TrkA in a similar way as in neuronal cells occurs. In LPS activated monocytes the block of TrkA with TrkA antibodies or TrkA chimera drastically reduces the phosphorylation of TrkA in the presence of NGF since it prevents NGF from TrkA binding (Fig. 4).

On the contrary the use of anti p75NTR antibodies or p75NTR chimera results in an increase of TrkA phosphorylation when NGF is added to culture medium. This demonstrates independently on the way by which the NGF and p75NTR binding is prevented, NGF anyway can bind TrkA, induce the phosphorylation of tyrosine residues and activate intracellular thereto correlated signal pathways. (Fig. 5).

Culture condition A): just isolated and quiescent monocytes express basal levels both of TrkA and p75-NTR. TrkA is expressed higher than p75-NTR (Fig1) and trkA/p75-NTR ratio being approximately 2. The stimulation with TLRs ligands induces remarkable increase of TrkA expression (Fig. 1) and low increase of p/75-NTR expression, TrkA/p75 ratio being approximately 4.

Culture condition B): monocytes are pre-incubated overnight (with NGF and an increase of p75-NTR expression and reduction of TrkA expression are observed (Fig. 6), respectively. Consistently with this opposite regulation of receptors, also signal pathways activated under said two conditions are different. When more p75NTR is expressed then NFk-B signal pathway is more activated (Fig.7), while, in the presence of elevated levels of TrkA, Akt regulated signal pathways are activated (Fig 8a and 8b), which are involved in the endogenous mechanisms of inflammation inhibition³⁰ and IkB phosphorylation (Fig 8c) and NF-kb nuclear translocation are inhibited (Fig 8d).

### Effects of NGF on cytokines synthesis.

The experiments have been carried out using two different experimental systems:
A) Condition: TLRs Stimulation and successive NGF addition Human monocytes have been stimulated with TLRs ligands and at the same time NGF is administered. The addition of NGF during TLR stimulation results in reduction of IL-6 production (Figure 9a). The addition of NGF neutralizing antibody together with NGF (aNGF+NGF) removes the NGF effect on IL-6 synthesis inhibition as observable after TLRs activation (Fig 9 A), thus demonstrating that the effect is NGF mediated. NGF reduces also the synthesis of other inflammatory cytokines such as IL-1β (Figure 9b), TNF-α, IL-8 (Fig. 9C) and this inhibition effect is NGF dose dependent (Figure 9d).
   Further NGF addition results in an increase of release of anti-inflammatory IL-10 cytokine and IL-1ra, that is an antagonist of IL-1 receptor (Fig 10, a,b). Also under these experimental conditions NGF alone does not modify the synthesis of inflammatory or anti-inflammatory cytokines in not stimulated monocytes.
B) Condition: Pre-Incubation with NGF and successive TLRs stimulation

Purified human monocytes are pre-incubated with NGF and successively stimulated with TLRs ligands. When stimulated with TLR2 (with PAM and LTA), TLR4 (with LPS) and TLR5 (with Flagellin), a NGF dose-dependent increase (fig. 11d) in the expression and release of IL-6 (Figure 11a), IL-1β (Figure 11b), IL-8 (figure 11c), is observed. In the absence of TLRs specific ligands NGF alone does not have any effect on the expression or release of cytokines in not stimulated monocytes.

### Effects of NGF receptor inhibition on cytokines synthesis.

In order to better discriminate the differences in signal pathways activated by either NGF receptors TrkA neutralizing monoclonal antibodies (goat polyclonal antibody obtained by immunization with extracellular domain of human recombinant Trka protein, R&D Systems) and p75NTR (monoclonal antibody, ME20.4 clone, Chemicon) and chimeric recombinant molecules consisting of human Fc fragment covalently linked to receptor extracellular portion (synthesized and commercially available from R&D Systems). These molecules represent in fact soluble receptors suitable to bind NGF, occurring in cellular culture, in p75-NTR or TrkA specific binding sites and thus preventing the binding thereof with cell occurring receptors.

Obtained data confirm results observed under two different culture conditions wherein a receptor was expressed in higher amount than the other and demonstrate the NGF ability to act in opposite way according to the type of available receptor.

In the presence of anti-TrkA antibodies or TrkA chimera, the NGF and TrkA binding is blocked and only the binding of NGF and p75NTR is possible. By blocking TrkA and inducing TLRs activation with ligands, the NGF addition results in a decrease of AKT phosphorylation and a consequent decrease of GSK3 inhibiting phosphorylation (Fig. 12a), an increase of IkB phosphorylation (Fig 12b) and higher NF-kB nuclear translocation (Fig 12c), an increase in the synthesis of inflammatory cytokines (Fig 12D) and a reduction of anti-inflammatory IL-10 cytokine (Fig 12E). On the contrary, by blocking the binding of NGF with p75NTR by monoclonal antibody or chimera or LM11A compounds, the binding of NGF occurs only with TrkA. By blocking p75NTR, NGF binds TrkA inducing phosphorylation thereof (Fig 5) and activation of intracellular pathways resulting in a decrease of the synthesis of inflammatory cytokines after TLRs activation (Fig 13a, b), and an increase of IL-10 synthesis (Fig 13c). This regulation occurs by activation of PI3K/AKT pathway and GSK3 inhibiting phosphorylation (Fig 13d) which seem to be key mechanisms to limit the inflammatory response and regulate the synthesis of IL-10 inhibiting in the same time the synthesis of inflammatory cytokines (18).

These data show that NGF, through TrkA, performs an anti-inflammatory role while if it acts through p75-NTR enhances the inflammatory response. Therefore it results that a dysregulation in the expression of NGF receptors could be involved in the onset of inflammatory diseases.

### MNC analyses and tissues from patients suffering from chronic inflammatory pathologies

In order to test the hypothesis that the alteration of TrkA/p75NTR ratio can contribute to the pathogeneses of inflammatory diseases resulting in dysregulation of the inflammatory response, we have analyzed the expression of NGF receptors and ratio thereof in mononuclear cells (MNC), from synovial liquids and blood of patients suffering from Juvenile Idiopathic Arthritis. Our data indicate that in patients suffering from Juvenile Idiopathic Arthritis (n=27) an increased expression of p75NTR and reduced expression of TrkA exist, while in healthy controls (n=16) the expression of TrkA is always higher than p75NTR. In patients therefore a ratio TrkA/p75NTR lower than 1, p75NTR (Fig 14 a), i.e. the receptor that by binding NGF is suitable to activate signal pathways inducing synthesis of inflammatory cytokines, being higher. It is to be pointed out that that in these patients a greater production of NGF in the inflamed tissues exists. Therefore these patients have an altered receptor ratio, p75NTR being higher, resulting in TrkA/p75NTR ratio lower than 1 and significant amounts of occurring NGF interacting preferentially with p75-NTR receptor, thus amplifying the local inflammatory response of native immune cells.

This TrkA decreased and p75NTR increased expressions determining an inversion of TrkA/p75NTR ratio seems to be a phenomenon shared also by other chronic inflammatory diseases as Rheumatoid Arthritis. The synovia of these patients are characterized by an elevated expression of p75NTR and a reduced expression of TrkA determining an alteration TrkA/p75NTR ratio (Figure 14b).

### Activity of p75NTR inhibitors on cytokines release in human synoviocytes from rheumatoid arthritis patients

Synoviocytes obtained from synovia of rheumatoid arthritis patients maintain during experiments ex vivo and in vitro the ability to secrete inflammatory cytokines in spontaneous way. We have estimated the expression of TrkA and p75NTR receptors in these cells. The expression of p75NTR is elevated and higher than TrkA (Fig15a), with expression ratio of TrKA/p75NTR receptors lower than 1. This expression imbalance of above said receptors is similar to what found within synovia of these patients. The in vitro p75NTR inhibitor addition resulted in a reduction of spontaneous synthesis of inflammatory cytokines in these patient cells (Fig15b), confirming the anti-inflammatory potential of p75NTR receptor blocking.

### BIBLIOGRAPHY

1) Zeytun A et al. Crit Rev Immunol. 2010;30:53-67.
2) Bracci-Laudiero L. Nerve Growth Factor and Inflammation. http://www.brainimmune.org , open access 15.9.2011;
3) Villoslada P et al. J Exp Med. 2000;191:1799-806
4) Arredondo LR et al. Eur J Immunol. 2001;31:625-33;
5) Flügel A et al. Eur J Immunol. 2001;31:11-22;
6) Micera A et al. J Neuroimmunol. 2000;104:116-23;
7) Gillardon F et al. Neuroreport. 1995;6:1322-4;
8) Townley SL et al.J Invest Dermatol. 2002;118:396-401;
9) Barada KA et al. Cytokine. 2007;37:236-45;
10) Reinshagen M et al. Gastroenterology. 2000;119:368-76;
11) Reichardt LF. Philos Trans R Soc Lond B Biol Sci. 2006 ; 361:1545-64;
12)Jiang Yet al. J Immunol. 2007;179:6297-304;
13)Jiang Y et al. Mol Immunol. 2008;45:1557-66;
14)Franklin RA et al. J Immunol. 1995;154:4965-72;
15)Wehrman T et al. Neuron. 2007;53:25-38;
16)Vilar M et al. Neuron. 2009; 62:72-83;
17)Micera A, et al. Mol Vis. 2007 ;13:981-7;
18)Han J and Ulevitch R. Nat Immunol. (2005; 6:1198-1205;
19)Wang H. et al. Cytokine (2011); 53:130-140;
20)Peleshok J, Saragovi HU. Biochem Soc Trans. 2006;34:612-7.
21)Lebrun-Julien F et al. Mol Cell Neurosci. 2009; 40:410-20.
22)Saragovi HU, et al. Curr Alzheimer Res. (2009)6:419-23.
23)Yaar M et al. Neuropathol Appl Neurobiol. (2007);33:533-43.
24)Yaar M et al. Cell Mol Neurobiol. (2008);28:1027-31.
25)Maliartchouk S et al. (2000) Mol Pharmacol.; 57:385-91.
26)Mahapatra S et al. (2009); J Biol Chem. 284:33600-13.
27)Bai Y et al. (2010) J Biol Chem. 285:39392-400;
28)Spiegel K. et al. Biochem Biophys Res Commun. (1995);217:488-94.
29)Colquhoun A et al. J Pharmacol Exp Ther. (2004);310:505-11.
30)Klinger MB. Et al. Am J Physiol Renal Physiol. (2008);295:F1778-89.
31)Jang SW. et al. Proc Natl Acad Sci USA. (2007) ;104:16329-34.
32)Chan CB. et al. Oncogene. (2009) ;28:3825-36.
33)Pehar M. et al. Eur J Neurosci. 2006 ;24:1575-80.
34)Massa SM. et al. J Neurosci. 2006 May 17;26(20):5288-300.
35)Yang T. et al. PLoS One. 2008;3:e3604.
36)Longo FM. et al. CNS Neurol Disord Drug Targets. 2008;7:63-70.
37)LeSauteur L. et al. J Neurosci. (1996);16:1308-16.
38)Petersen et al., Neuroscience Vol. 83, No. 1, pp. 161-168, 1998.
39)Barthel C et al. Arthritis Research & Therapy 2009, 11:R82

## Claims

1. Compound selected from inhibitors of p75NTR receptor, alone or in association with at least one compound selected from TrkA receptor activators, or a compound selected from TrkA receptor activators, for use in anti-inflammatory treatment of the chronic inflammatory diseases of auto-inflammatory or autoimmune origin, with the exclusion of allergic origin diseases, in patients wherein the expression ratio TrkA receptor/p75NTR receptor is lower than 1 in inflamed tissues or cells of biological fluids;
wherein the inhibitors of p75NTR receptor are selected from the group consisting of polyclonal and monoclonal monospecific or bispecific p75NTR receptor binding antibodies, chimerical molecules whose structure includes soluble p75NTR receptor, p75NTR receptor binding cyclic monomeric and dimeric NGF analogous compounds and wherein the TrkA receptor activators are selected from the group consisting of TrkA receptor binding polyclonal and monoclonal antibodies, cyclic monomeric and dimeric NGF analogous compound, recombinant NGF or recombinant mutated NGF.

2. Compound for use according to claim 1, wherein p75NTR receptor binding monoclonal antibodies are selected from the group consisting of anti-nerve growth factor receptor p75 human clone ME20.4 monoclonal antibody, MLR-1, MLR2, MLR3 monoclonal antibodies, anti-p75NTR receptor human clone HB-8737 antibody.

3. Compound for use according to claim 1, wherein chimerical molecule whose structure includes soluble p75NTR receptor is NGFR/TNFRSF16/Fc.

4. Compound for use according to claim 1, wherein polyclonal antibody is selected among anti-p75NTR receptor neutralizing polyclonal antibody (REX), rabbit 9651 or 9650 polyclonal antibodies.

5. Compound for use according to claim 1, wherein p75NTR receptor binding cyclic monomeric and dimeric NGF analogous compounds are selected among NGF C30-35, PD90780 compound, LM11A compounds.

6. Compound for use according to claim 5, wherein LM11A compounds are selected from the group consisting of LM11A-24 caffeine or LM11A-31 isoleucine derivative compounds.

7. Compound for use according to claim 1, wherein TrkA receptor binding monoclonal antibody is 5C3.

8. Compound for use according to claim 1, wherein NGF cyclic analogous compound is NGF C92-97.

9. Compound for use according to claim 1, wherein recombinant mutated NGF is NGFKKE/7-84-103.

10. Compound for use according to claim 1, wherein TrkA receptor activators are selected among D3 peptide, gambogic amide, 3-aza-bicyclo [3.2.1] octane derivatives.

11. Compound for use according to anyone of the preceding claims, wherein said chronic inflammatory diseases are selected from the group consisting of rheumatoid arthritis, juvenile idiopathic arthritis, intestinal chronic inflammatory diseases, ankylosing spondylitis, psoriasis, multiple sclerosis, Lupus erythematosus, scleroderma, Syndrome of Sjogren.

12. Compound for use according to claim 11, wherein the intestinal chronic inflammatory diseases are selected from Crohn's Disease, ulcerous colitis.

13. Combination of at least one inhibiting compound of p75NTR receptor with at least one activator of TrkA receptor for the separated or sequential use in anti-inflammatory treatment of chronic inflammatory diseases of auto-inflammatory or autoimmune origin, with the exclusion of allergic origin diseases, in patients wherein the expression ratio TrkA receptor/p75NTR receptor is lower than 1 in inflamed tissues or cells of biological fluids; wherein the inhibitors of p75NTR receptor are selected from the group consisting of polyclonal and monoclonal monospecific or bispecific p75NTR receptor binding antibodies, chimerical molecules whose structure includes soluble p75NTR receptor, p75NTR receptor binding cyclic monomeric and dimeric NGF analogous compounds and wherein the TrkA receptor activators are selected from the group consisting of TrkA receptor binding polyclonal and monoclonal antibodies, cyclic monomeric and dimeric NGF analogous compound, NGF or recombinant NGF or recombinant mutated NGF.

14. Combination for use according to claim 13, wherein said chronic inflammatory diseases are selected from the group consisting of rheumatoid arthritis, juvenile idiopathic arthritis, intestinal chronic inflammatory diseases, ankylosing spondylitis, psoriasis, multiple sclerosis, Lupus erythematosus, scleroderma, Syndrome of Sjogren.

15. Combination for use according to claim 13, wherein the intestinal chronic inflammatory diseases are selected from Crohn's Disease, ulcerous colitis.

16. In vitro screening method to evaluate the anti-inflammatory effect of pharmacological inhibitors of p75NTR receptor in chronic inflammatory diseases of auto-inflammatory or autoimmune origin, said method comprising or consisting of the following steps: a) contacting isolated tissues or mononucleated cells of a patient affected by chronic inflammatory disease with said inhibitor; b) measuring the levels of pro-inflammatory cytokines, released spontaneously or after stimulation with TLR ligands, wherein said chronic inflammatory diseases are selected from the group consisting of rheumatoid arthritis, juvenile idiopathic arthritis, intestinal chronic inflammatory diseases, ankylosing spondylitis, psoriasis, multiple sclerosis, Lupus erythematosus, scleroderma, Syndrome of Sjogren.

## Patentansprüche

1. Verbindung, ausgewählt aus Hemmern des p75NTR-Rezeptors, allein oder im Zusammenhang mit mindestens einer Verbindung, ausgewählt aus TrkA-Rezeptoraktivatoren, oder einer Verbindung, ausgewählt aus TrkA-Rezeptoraktivatoren, zur Verwendung bei der entzündungshemmenden Behandlung der chronischen Entzündungskrankheiten von auto-entzündlichem oder autoimmunem Ursprung, unter Ausschluss von Krankheiten von allergischem Ursprung, bei Patienten, worin das Expressionsverhältnis TrkA-Rezeptor/p75NTR-Rezeptor niedriger als 1 in entzündeten Geweben oder Zellen biologischer Fluids ist;
wobei die Hemmer vom p75NTR-Rezeptor ausgewählt sind aus der Gruppe, bestehend aus polyklonalen und monoklonalen monospezifischen oder bispezifischen p75NTR-Rezeptorbindungsantikörpern, chimären Molekülen, deren Struktur den löslichen p75NTR-Rezeptor beinhaltet, p75NTR-Rezeptor-bindende, cyclische, monomere und dimere NGF-analoge Verbindungen, und wobei die TrkA-Rezeptoraktivatoren ausgewählt sind aus der Gruppe, bestehend aus TrkA-Rezeptor-bindenden polyklonalen und monoklonalen Antikörpern, cyclischer monomerer und dimerer NGF-analoger Verbindung, rekombinantem NGF oder rekombinantem mutiertem NGF.

2. Verbindung zur Verwendung nach Anspruch 1, wobei p75NTR-Rezeptor-bindende monoklonale Antikörper ausgewählt sind aus der Gruppe, bestehend aus dem Anti-Nerven-Wachstumsfaktor-Rezeptor p75 humanen Klon ME20.4 monoklonalen Antikörper, MLR-1, MLR2, MLR3 monoklonalen Antikörpern, anti-p75NTR-Rezeptor humanem Klon HB-8737 Antikörper.

3. Verbindung zur Verwendung nach Anspruch 1, wobei ein chimäres Molekül, dessen Struktur den löslichen p75NTR-Rezeptor beinhaltet, NGFR/TNFRSF16/Fc ist.

4. Verbindung zur Verwendung nach Anspruch 1, wobei der polyklonale Antikörper ausgewählt ist aus anti-p75NTR-Rezeptor neutralisierendem polyklonalem Antikörper (REX), Kaninchen 9651 oder 9650 polyklonalen Antikörpern.

5. Verbindung zur Verwendung nach Anspruch 1, wobei p75NTR-Rezeptor-bindende, cyclische, monomere und dimere NGF-analoge Verbindungen ausgewählt sind aus NGF C30-35, PD90780-Verbindung, LM11A-Verbindungen.

6. Verbindung zur Verwendung nach Anspruch 5, wobei LM11A-Verbindungen ausgewählt sind aus der Gruppe, bestehend aus LM11A-24-Koffein oder LM11A-31-Isoleucinderivatverbindungen.

7. Verbindung zur Verwendung nach Anspruch 1, wobei TrkA-Rezeptor-bindender monoklonaler Antikörper 5C3 ist.

8. Verbindung zur Verwendung nach Anspruch 1, wobei die cyclische analoge NGF-Verbindung NGF C92-97 ist.

9. Verbindung zur Verwendung nach Anspruch 1, wobei rekombinantes mutiertes NGF NGFKKE/7-84-103 ist.

10. Verbindung zur Verwendung nach Anspruch 1, wobei TrkA-Rezeptoraktivatoren ausgewählt sind aus D3-Peptid, Gambogamid, 3-Aza-bicyclo[3.2.1]octanderivaten.

11. Verbindung zur Verwendung nach einem der vorstehenden Ansprüche, wobei die chronischen entzündlichen Erkrankungen ausgewählt sind aus der Gruppe, bestehend aus rheumatoider Arthritis, juveniler idiopathischer Arthritis, chronischen entzündlichen Darmerkrankungen, Morbus Bechterew, Psoriasis, Multipler Sklerose, Lupus erythematodes, Sklerodermie, Sjögren-Syndrom.

12. Verbindung zur Verwendung nach Anspruch 11, wobei die chronischen entzündlichen Darmerkrankungen ausgewählt sind aus Morbus Crohn, Colitis ulcerosa.

13. Kombination mindestens einer hemmenden Verbindung des p75NTR-Rezeptors mit mindestens einem Aktivator des TrkA-Rezeptors zur getrennten oder aufeinanderfolgenden Verwendung bei der entzündungshemmenden Behandlung von chronischen entzündlichen Erkrankungen von auto-entzündlichem oder autoimmunem Ursprung, unter Ausschluss von Erkrankungen von allergischem Ursprung, bei Patienten, worin das Expressionsverhältnis TrkA-Rezeptor/p75NTR-Rezeptor in entzündeten Geweben oder Zellen biologischer Fluids kleiner als 1 ist; wobei die Hemmer des p75NTR-Rezeptors ausgewählt sind aus der Gruppe, bestehend aus polyklonalen und monoklonalen monospezifischen oder bispezifischen p75NTR-Rezeptorbindungsantikörpern, chimären Molekülen, deren Struktur den löslichen p75NTR-Rezeptor beinhaltet, p75NTR-Rezeptor-bindenden cyclischen monomeren und dimeren NGF-analogen Verbindungen und wobei die TrkA-Rezeptoraktivatoren ausgewählt sind aus der Gruppe, bestehend aus TrkA-Rezeptor-bindenden polyklonalen und monoklonalen Antikörpern, cyclischer monomerer und dimerer NGF-analoger Verbindung, NGF oder rekombinantem NGF oder rekombinantem mutiertem NGF.

14. Kombination zur Verwendung nach Anspruch 13, wobei die chronischen entzündlichen Erkrankungen ausgewählt sind aus der Gruppe, bestehend aus rheumatoider Arthritis, juveniler idiopathischer Arthritis, intestinalen chronischen entzündlichen Erkrankungen, Morbus Bechterew, Psoriasis, Multipler Sklerose, Lupus erythematodes, Sklerodermie, Sjögren-Syndrom.

15. Kombination zur Verwendung nach Anspruch 13, wobei die chronischen entzündlichen Darmerkrankungen ausgewählt sind aus Morbus Crohn, Colitis ulcerosa.

16. In vitro-Screeningverfahren zur Beurteilung der entzündungshemmenden Wirkung von pharmakologischen Hemmern des p75NTR-Rezeptors bei chronisch entzündlichen Erkrankungen von auto-entzündlichem oder autoimmunem Ursprung, wobei das Verfahren die folgenden Schritte umfasst oder aus diesen besteht: a) Kontaktieren von isolierten Geweben oder mononukleierten Zellen eines Patienten, der von einer chronischen entzündlichen Erkrankung betroffen ist, mit dem Hemmer; b) Messen des Spiegels von proentzündlichen Zytokinen, die spontan oder nach Stimulation mit TLR-Liganden freigesetzt werden, wobei die chronischen entzündlichen Erkrankungen ausgewählt sind aus der Gruppe, bestehend aus rheumatoider Arthritis, juveniler idiopathischer Arthritis, chronischen entzündlichen Darmerkrankungen, Morbus Bechterew, Psoriasis, Multipler Sklerose, Lupus erythematodes, Sklerodermie, Sjögren-Syndrom.

## Revendications

1. Composé sélectionné parmi les inhibiteurs du récepteur p75NTR, seul ou en association avec au moins un composé sélectionné parmi les activateurs du récepteur TrkA, ou un composé sélectionné parmi les activateurs du récepteur TrkA, pour son utilisation dans le traitement anti-inflammatoire des maladies inflammatoires chroniques d'origine auto-inflammatoire ou auto-immune, à l'exclusion des maladies d'origine allergique, chez les patients pour lesquels le rapport d'expression récepteur TrkA/récepteur p75NTR est inférieur à 1 dans les tissus ou les cellules de fluides biologiques inflammés ;
dans lesquels les inhibiteurs du récepteur p75NTR sont sélectionnés dans le groupe constitué par les anticorps de liaison au récepteur p75NTR monospécifiques ou bispécifiques polyclonaux et monoclonaux, les molécules chimériques dont la structure comprend le récepteur soluble p75NTR, les composés analogues du NGF dimères et monomères cycliques de liaison au récepteur p75NTR et dans lesquels les activateurs du récepteur TrkA sont sélectionnés dans le groupe constitué par les anticorps polyclonaux et monoclonaux de liaison au récepteur TrkA, les composés analogues du NGF dimères et monomères cycliques, le NGF recombinant ou le NGF muté recombinant.

2. Composé pour son utilisation selon la revendication 1, dans lequel les anticorps monoclonaux de liaison au récepteur p75NTR sont sélectionnés dans le groupe constitué par les anticorps monoclonaux du récepteur du facteur de croissance nerveuse p75 clone humain ME20.4, MLR-1, MLR2, anticorps monoclonaux, anticorps HB-8737 clone humain récepteur anti-p75NTR.

3. Composé pour son utilisation selon la revendication 1, dans lequel la molécule chimérique dont la structure comprend le récepteur soluble de p75NTR est NGFR/TNFRSF16/Fc.

4. Composé pour son utilisation selon la revendication 1, dans lequel l'anticorps polyclonal est sélectionné parmi l'anticorps (REX) polyclonal de neutralisation du récepteur anti-p75NTR, les anticorps polyclonaux de lapin 9651 ou 9650.

5. Composé pour son utilisation selon la revendication 1, dans lequel les composés analogues du NGF dimères et monomères cycliques de liaison au récepteur p75NTR sont sélectionnés parmi les composés NGF C30-35, PD90780, LM11A.

6. Composé pour son utilisation selon la revendication 5, dans lequel les composés LM11A sont sélectionnés dans le groupe constitué des dérivés de caféine LM11A-24 ou d'isoleucine LM11A-31.

7. Composé pour son utilisation selon la revendication 1, dans lequel l'anticorps monoclonal de liaison au récepteur TrkA est 5C3.

8. Composé pour son utilisation selon la revendication 1, dans lequel le composé analogue cyclique du NGF est NGF C92-97.

9. Composé pour son utilisation selon la revendication 1, dans lequel le NGF muté recombinant est NGFKKE/7-84-103.

10. Composé pour son utilisation selon la revendication 1, dans lequel les activateurs du récepteur TrkA sont sélectionnés parmi les dérivés du peptide D3, de l'amide gambogique, du 3-aza-bicyclo [3.2.1] octane.

11. Composé pour son utilisation selon l'une quelconque des revendications précédentes, dans lequel lesdites maladies inflammatoires chroniques sont sélectionnées dans le groupe constitué par la polyarthrite rhumatoïde, l'arthrite idiopathique juvénile, les maladies inflammatoires chroniques intestinales, la spondylarthrite ankylosante, le psoriasis, la sclérose en plaques, le lupus érythémateux, la sclérodermie, le syndrome de Sjogren.

12. Composé pour son utilisation selon la revendication 11, dans lequel les maladies inflammatoires chroniques intestinales sont sélectionnées parmi la maladie de Crohn, la colite ulcéreuse.

13. Combinaison d'au moins un composé inhibiteur du récepteur p75NTR avec au moins un activateur du récepteur TrkA pour son utilisation séparée ou séquentielle dans le traitement anti-inflammatoire des maladies inflammatoires chroniques d'origine auto-inflammatoire ou auto-immune, à l'exclusion des maladies d'origine allergique, chez les patients pour lesquels le rapport d'expression récepteur TrkA/récepteur p75NTR est inférieur à 1 dans les tissus ou les cellules de fluides biologiques inflammés ; dans lesquels les inhibiteurs du récepteur p75NTR sont sélectionnés dans le groupe constitué par les anticorps de liaison au récepteur p75NTR monospécifiques ou bispécifiques polyclonaux et monoclonaux, les molécules chimériques dont la structure comprend le récepteur p75NTR soluble, les composés analogues du NGF dimères et monomères cycliques de liaison au récepteur p75NTR et dans lesquels les activateurs du récepteur TrkA sont sélectionnés dans le groupe constitué par les anticorps polyclonaux et monoclonaux de liaison au récepteur TrkA, les composés analogues du NGF dimères et monomères cycliques, le NGF recombinant ou le NGF muté recombinant.

14. Combinaison pour son utilisation selon la revendication 13, dans laquelle lesdites maladies inflammatoires chroniques sont sélectionnées dans le groupe constitué par la polyarthrite rhumatoïde, l'arthrite idiopathique juvénile, les maladies inflammatoires chroniques intestinales, la spondylarthrite ankylosante, le psoriasis, la sclérose en plaques, le lupus érythémateux, la sclérodermie, le syndrome de Sjogren.

15. Combinaison pour son utilisation selon la revendication 13, dans laquelle les maladies inflammatoires chroniques intestinales sont sélectionnées parmi la maladie de Crohn, la colite ulcéreuse.

16. Méthode de criblage in vitro pour évaluer l'effet anti-inflammatoire d'inhibiteurs pharmacologiques du récepteur p75NTR dans des maladies anti-inflammatoires chroniques d'origine auto-inflammatoire ou auto-immune, ladite méthode comprenant ou consistant en les étapes suivantes: a) à mettre en contact des tissus isolés ou des cellules mononucléées d'un patient atteint d'une maladie inflammatoire chronique avec ledit inhibiteur; b) à mesurer les niveaux de cytokines pro-inflammatoires, libérées spontanément ou après stimulation avec des ligands de TLR, lesdites maladies inflammatoires chroniques étant sélectionnées dans le groupe constitué de la polyarthrite rhumatoïde, l'arthrite idiopathique juvénile, les maladies inflammatoires chroniques intestinales, la spondylarthrite ankylosante, le psoriasis, la sclérose en plaques, le lupus érythémateux, la sclérodermie, le syndrome de Sjogren.
